# EUROPEAN PATENT SPECIFICATION

(11) **EP 1 818 392 B1**
(45) Date of publication and mention of the grant of the patent: **25.08.2010**
(21) Application number: 07250470.7
(22) Date of filing: 05.02.2007
(51) Int. Cl.: C12N 5/07

(54) **Cell culture medium**
Zellkulturmedium
Support de culture de cellules

(30) Priority: 14.02.2006 US 772862 P
(43) Date of publication of application: 15.08.2007
(73) Proprietor: Genetix Limited, New Milton, Hampshire BH25 5NN (GB)
(72) Inventor: Bramke, Irene, c/o Genetix Limited, New Milton Hampshire BH25 5NN (GB); Burke, Julian Francis, c/o Genetix Limited, New Milton Hampshire BH25 5NN (GB)
(74) Representative: Harding, Charles Thomas

(56) References cited:
- EP-A1- 0 780 469
- WO-A-96/01426
- US-A- 5 073 492
- US-A- 5 122 469
- URLAUB G ET AL: "ISOLATION OF CHINESE HAMSTER CELL MUTANTS DEFICIENT IN DIHYDROFOLATE REDUCTASE ACTIVITY" PROCEEDINGS OF THE NATIONAL ACADEMY OF SCIENCES OF THE UNITED STATES, NATIONAL ACADEMY OF SCIENCE, WASHINGTON, DC, US, vol. 77, no. 7, July 1980 (1980-07), pages 4216-4220, XP008004784 ISSN: 0027-8424
- CHEN S C ET AL: "PURIFICATION AND CHARACTERIZATION OF 2 VASCULAR ENDOTHELIUM EFFECTORS FROM FETAL BOVINE RETINA VITREOUS AND SERUM" EXPERIMENTAL EYE RESEARCH, vol. 41, no. 1, 1985, pages 77-86, XP002438195 ISSN: 0014-4835
- HOU MINGYAN ET AL: "UDP acts as a growth factor for vascular smooth muscle cells by activation of P2Y6 receptors" AMERICAN JOURNAL OF PHYSIOLOGY, vol. 282, no. 2 Part 2, February 2002 (2002-02), pages H784-H792, XP002438196 ISSN: 0002-9513
- HOLEN ET AL: "Dietary nucleotides and human immune cells. II. Modulation of PBMC growth and cytokine secretion" NUTRITION, NUTRITION, BURBANK, CA, US, vol. 22, no. 1, January 2006 (2006-01), pages 90-96, XP005271196 ISSN: 0899-9007
- BHAGWAT SS WILLIAMS M: "P2 purine and pyrimidine receptors: emerging superfamilies of G-protein-coupled and ligand-gated ion channel receptors" EUROPEAN JOURNAL OF MEDICINAL CHEMISTRY, EDITIONS SCIENTIFIQUE ELSEVIER, PARIS, FR, vol. 32, no. 3, 1997, pages 183-193, XP004075420 ISSN: 0223-5234

## Description

### FIELD

This invention relates to the field of cell biology and biotechnology, and in particular, *in vitro* cell culture.

### BACKGROUND

Animal cells are cultured *in vitro* for various purposes, for example to obtain a natural protein produced by the animal cell, or in the production of recombinant proteins from an animal cell incorporating a gene coding for the desired protein. Different cell types often have individual nutritional requirements and therefore, many distinct media have been developed specifically to meet the nutritional requirement of the different cell types. In general, the culture medium includes essential nutrients, such as bases, sugars, amino acids, and vitamins.

In addition to the essential nutrients, many of these media contain sera, which is essentially a mammal-derived extract, as an additive, such as FBS (fetal bovine serum), HS (horse serum) and CS (calf serum). Serum is usually added to the medium a range of 5 to 20% in order to promote proliferation of the animal cell.

In culturing an animal cell, the failure to add serum into the culture medium causes a marked drop in the survival rate of cells, and a decrease in viable cell count in the culture broth, at an early stage of culture. These events make long-term culture or large-scale culture impossible.

However, such serum has a number of drawbacks. It accounts for 75 to 95% of the cost for the culture medium, and because of inter-lot differences existing in quality, stable proliferation is not achieved. Additionally, serum contains growth-inhibitory and differentiating factors, as well as de-stabilizing the genetic material leading to genetic changes and eventually senescence (Loo et al., Science 236 (4798):200-2 (Apr. 10, 1987)) of the cells. Furthermore, the serum contains more than 500 types of proteins, thus complicating the isolation and purification of the desired protein, the cell product, from the cultured medium.

Importantly from the point of view of health and safety, the serum cannot be sterilized in an autoclave or the like, and thus may be contaminated with viruses or mycoplasmas. Although most of these viruses or mycoplasmas are nonpathogenic, they can become additional unknown factors from the point of view of stable manufacture. In recent years, however, concern has been expressed over the relation of mammal-derived components to mad cow disease, bovine spongiform encephalopathy (BSE), transmissible spongiform encephalopathy (TSE), and Creutzfeld-Jakob disease (CJD). The absence of animal derived proteins is desirable from a regulatory standpoint, and may be required as part of good medical practice (GMP) compliance.

These safety concerns have led to the demand for serum-free cell culture media to be developed. For example, CD CHO Medium (Invitrogen) and HyQ CDM4CHO, HyQ® SFM4CHO and HyQ® SFM4CHO™-Utility (HyClone, Logan, Utah, USA) are chemically-defined media suitable for the growth of Chinese Hamster Ovary (CHO) cells and expression of recombinant proteins in suspension culture. However, it has been found that CHO cells don't grow as well in chemically defined serum-free media as they do in serum-containing media.

There is therefore a need in the art for a cell culture medium which enables animal cells to grow at a suitable rate, but without relying on the presence of serum.

Furthermore, it is often desirable to increase the throughput by automating the plating and colony picking processes, for example by the use of robotic devices such as the ClonePix and ClonePix FL devices (Genetix, New Milton, United Kingdom), described in US 11/050,826, 11/050,818 and 11/199,243, herein incorporated by reference. Such colony pickers will typically make use of animal cells growing in semi-solid media such as methylcellulose media. However, a significant problem exists in that the rate of growth of CHO cells on such semi-solid media declines further when the media does not contain serum.

Carvalhal and colleagues describe the use of cell growth arrest by nucleotides, nucleosides and bases as a tool for improved production of recombinant proteins. Carvalhal et al exposed CHO cells expressing human placental alkaline phosphatase (SEAP) to purines and pyrimidines, in a FMX-8 medium supplemented with10% foetal bovine serum (FBS), 4.5 g/l of glucose, 4 mM glutamine, 5 mg/l of puromycin and 400 mg/l of G418. A number of purines were seen to cause cell growth arrest. In general, the pyrimidines did not have a significant effect on cell growth compared to the control.

Based on these results, Carvalhal et al propose the use of adenosine monophosphate in arresting cell growth for increasing production of recombinant proteins. This work is described in Carvalhal et al (2003) Biotechnol Prog. 19(1):69-83; Carvalhal et al. (2003) Biotechnol Prog 19:69-83; Sukhorukov et al (1994) J Membr Biol 142:77-92; Al-Rubeai et al (1992) Cytotechnology 9:85-97; Carvalhal et al (2002) Enz Microb Technol 30(1):95-109; Mazur et al (1998). Biotechnol Prog 14(5): 705-13; and Cruz et al (1997) Biotech Tech 11(2): 117-120.

### SUMMARY

The present invention aims at providing a culture medium for culture of an animal cell, the culture medium containing no mammal-derived components and freed from the above problems, and a method for producing a protein with the use of the culture medium.

Contrary to the findings of Carvalhal and colleagues, we have demonstrated that pyrimidines, including thymidine, uridine analogues thereof and other thymidine family members have a stimulatory activity on cell growth and viability. We therefore propose the use of increased concentrations of such substances in cell culture media to prolong the life of, prevent the death of, and increase protein yield from, cultured animal cells.

We provide a method of growing an animal cell in a culture medium, in which the culture medium comprises an elevated concentration of a thymidine family member, in which the growth or viability of the animal cell is increased as a result of the elevated concentration of the thymidine family member in the cell culture medium.

The elevated concentration of the thymidine family member in the cell culture medium is between 50 µg/l to about 50 mg/l.

The method comprises supplementing a basal medium with a supplement comprising the thymidine family member to produce the cell culture medium. The supplement may comprise a 50x supplement and may comprise 2.5 mg/l to about 2500mg/l of the thymidine family member. The basal medium may comprise a minimal medium. Preferably, the basal medium substantially lacks the thymidine family member.

The cell culture medium is substantially free of serum. The cell culture medium may comprise a chemically defined medium.

The thymidine family member is selected from the group consisting of: thymidine, a thymidine analogue, uridine and a uridine analogue. In some embodiments, the cell culture medium comprises an elevated concentration of both thymidine and uridine.

The cell culture medium comprises thymidine at about 0.24 mg/l. Alternatively, or in addition, the cell culture medium comprises uridine at 7 mg/ml. Preferably, the cell culture medium does not substantially comprise adenosine.

Preferably, the cell culture medium further comprises conditioned medium.

In some embodiments, the cell culture medium is a semi-solid medium. In such embodiments, the semi-solid medium preferably comprises methylcellulose. In other embodiments, the cell culture medium is a liquid medium.

Preferably, cell growth or cell viability is enhanced by at least 50% compared to growth in a cell culture medium without an elevated concentration of thymidine family member.

Preferably, apoptosis of the animal cell in the cell culture medium is reduced to enhance cell viability.

In some embodiments, the animal cell is transformed with an expression vector, the expression vector being a member of a nucleic acid library.

The animal cell may express a heterologous or recombinant protein. The heterologous or recombinant protein may be a therapeutic protein or a therapeutic antibody.

Preferably, the animal cell comprises an animal cell line.

The animal cell may comprise a rodent cell. The animal cell is preferably selected from the group consisting of: Chinese Hamster Ovary (CHO) cell and CHO-S cell.

There is provided use of a thymidine family member to enhance the growth or viability of a Chinese Hamster Ovary (CHO) cell in a semi-solid serum-free culture medium.

We provide, according to an aspect of the present invention, method of producing a recombinant protein, the method comprising culturing an animal cell which expresses the recombinant protein in a method according to the invention.

As an aspect of the present invention, there is provided a method of increasing the yield of a recombinant protein from an animal cell, the method comprising enhancing the growth or viability of the animal cell by a method according to the invention.

Preferably, the yield is increased by at least 10% compared to the yield from a culture medium without an elevated concentration of thymidine family member.

We provide a cell culture medium comprising a growth enhancing concentration of a thymidine family member.

The present invention provides a composition comprising 350 mg/l of uridine and 12 mg/l of thymidine.

There is provided a method of producing a recombinant protein, the method comprising culturing an animal cell which expresses the recombinant protein in a liquid medium comprising an elevated concentration of a thymidine family member.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual : Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855, Lars-Inge Larsson "Immunocytochemistry: Theory and Practice", CRC Press inc., Baca Raton, Florida, 1988, ISBN 0-8493-6078-1, John D. Pound (ed); "Immunochemical Protocols, vol 80", in the series: "Methods in Molecular Biology", Humana Press, Totowa, New Jersey, 1998, ISBN 0-89603-493-3, Handbook of Drug Screening, edited by Ramakrishna Seethala, Prabhavathi B. Fernandes (2001, New York, NY, Marcel Dekker, ISBN 0-8247-0562-9); Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3; and The Merck Manual of Diagnosis and Therapy (17th Edition, Beers, M. H., and Berkow, R, Eds, ISBN: 0911910107, John Wiley & Sons). Each of these general texts is herein incorporated by reference.

### BRIEF DESCRIPTION OF THE DRAWINGS

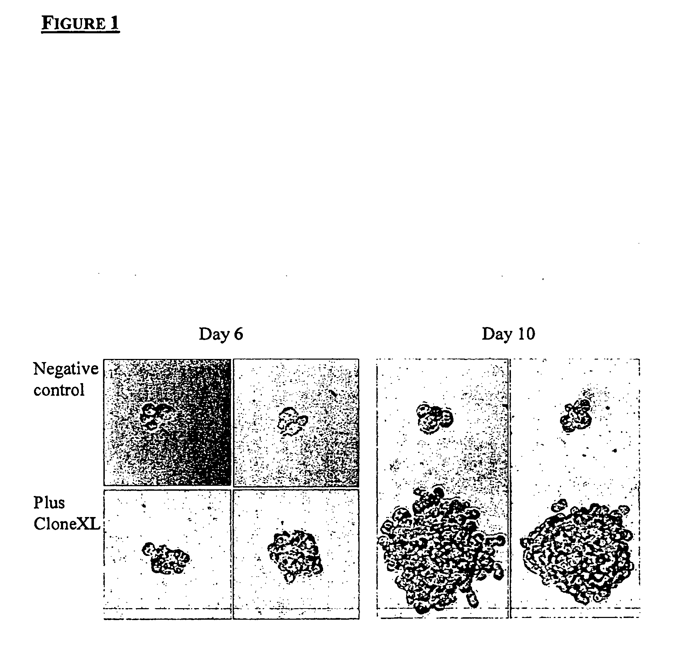

Figure 1 shows Images of typical colonies, grown in the absence (top row) and presence (bottom row) of CloneXL Supplement. Images are taken on day 6 and day 10 post-plating at 40-fold magnification.

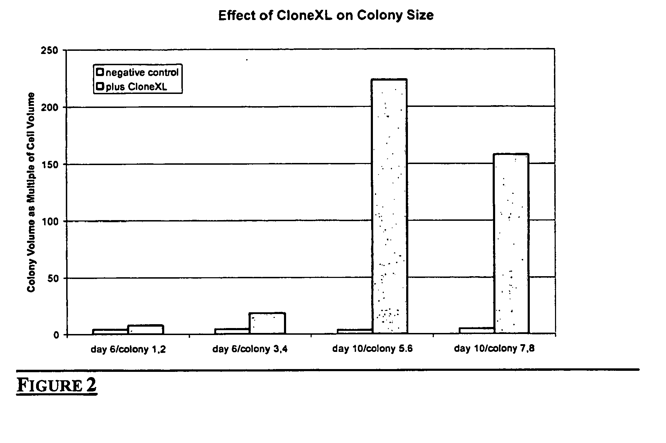

Figure 2 is a bar chart of volumes of typical colonies grown in the presence (white bars) and absence (grey bars) of CloneXL Supplement.

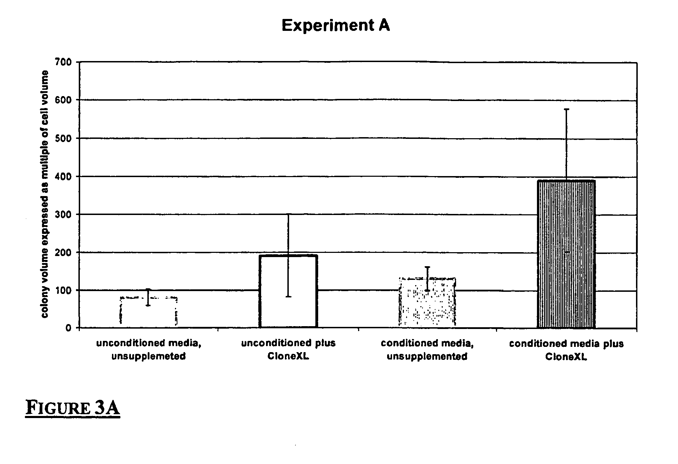

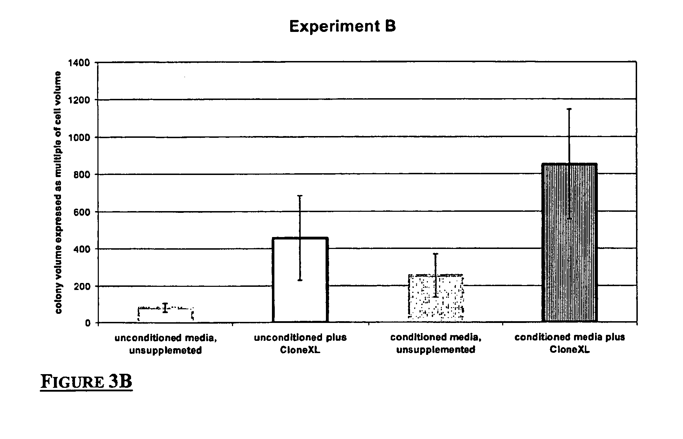

Figure 3 shows bar charts of average colony volumes for two duplicate experiments. Experiment A (top chart, data recorded for 4 colonies per condition) and Experiment B (bottom chart, data recorded for 3 colonies per condition). Colonies are grown in the absence (bars framed in grey) and presence (bars framed in red) of CloneXL Supplement containing thymidine and uridine, in unconditioned and 20% conditioned (shaded bars on the right) media. Colony volumes were derived from diameters recorded on day 6 post-plating and displayed as multiples of the volume of a cell. Error bars represent the standard deviation of the mean.

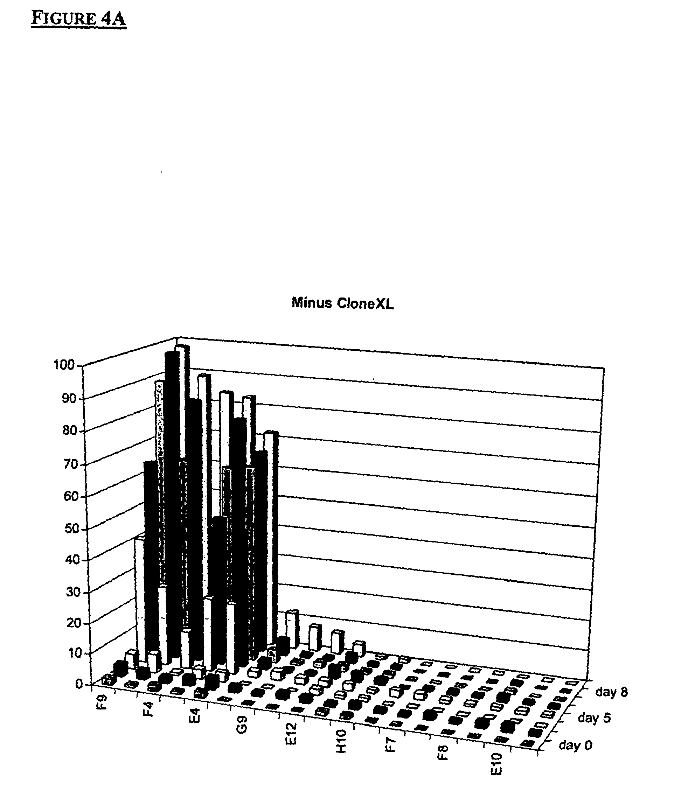

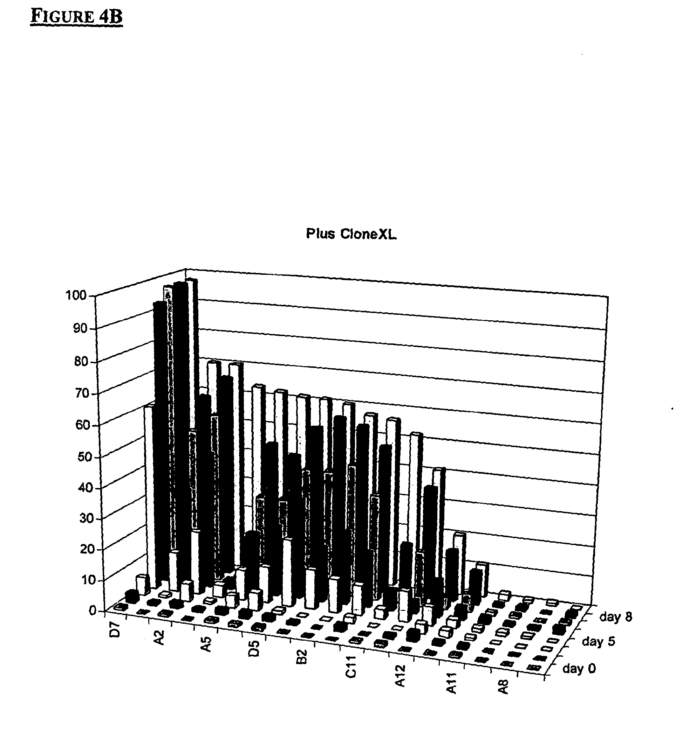

Figure 4A and 4B show bar charts of percentage confluence over time. Figure 4A: data recorded for 18 wells of the destination plate not supplemented with CloneXL (thymidine and uridine). Figure 4B: data recorded for data recorded for 18 wells of the destination plate supplemented with CloneXL (thymidine and uridine). Individual wells are ordered along the X-axis by decreasing level of confluence reached by day 8 post-picking. Confluence readings (Y-axis) are obtained using the CloneSelect Imager (Genetix) immediately after picking on Clone FL Mammalian Colony Picker (Genetix) and after 1, 2, 5, 6, 7, and 8 days of incubation.

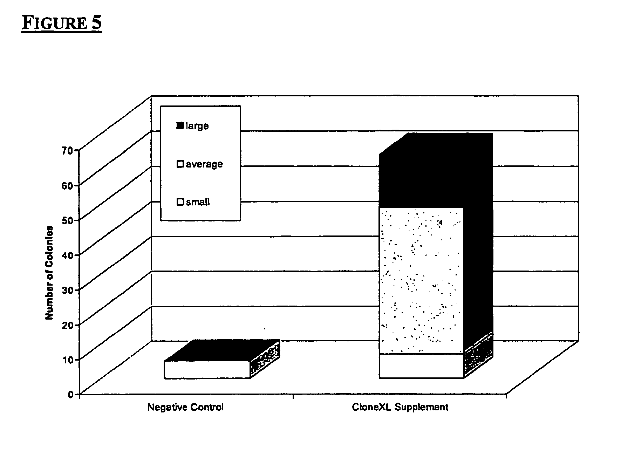

Figure 5 shows a bar chart of colony number. Left Bar, number of colonies obtained in 6 wells of cells plated in un-supplemented semisolid media. Right, number of colonies obtained in 6 wells of cells plated in semisolid media plus CloneXL Supplement

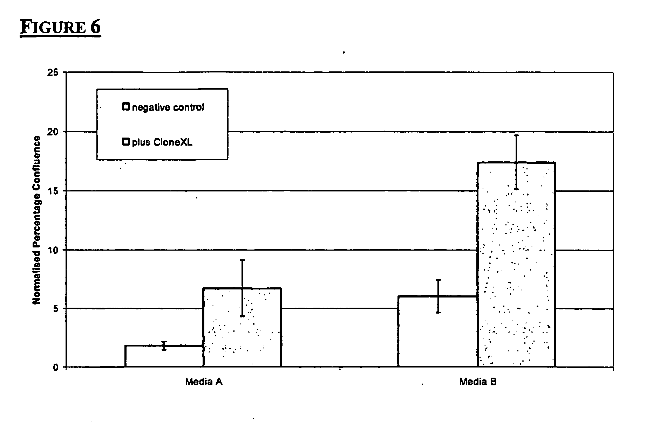

Figure 6 is a bar chart showing the effect of CloneXL on cell growth in liquid cultures of suspension-adapted CHO cells in two different commercially available chemically-defined CHO media. The left two bars show average normalised confluence readings obtained for un-supplemented (white) and CloneXL supplemented (grey) Media A. The left right bars showing average normalised confluence readings obtained for un-supplemented (white) and CloneXL supplemented (grey) Media B. Error bars denote the standard deviation from the mean of readings obtained for 48 independent cultures. Bottom two bars showing counts of live (white) and dead (grey) cells observed on day 19 of the second of two repeats of the experiment.

### DETAILED DESCRIPTION

### IMPROVED CELL CULTURE MEDIUM

We disclose an improved cell culture medium suitable for growing animal cells, comprising a thymidine family member. The thymidine family member is preferably at an elevated or increased concentration in the medium. The presence or addition of an elevated concentration of thymidine family member stimulates the growth of the animal cell. Thus, it enables an increase in cell viability and/or cell growth and/or a reduction in cell death (preferably apoptosis).

For convenience, the cell culture medium described herein comprising thymidine family member, preferably with an elevated concentration of thymidine family member, is referred to as an "improved cell culture medium". The nature of the "thymidine family member" is set out in further detail below.

Thus, we provide a method of enhancing the growth or viability of an animal cell in a culture medium, the method comprising culturing the animal cell in a cell culture medium comprising an elevated concentration of a thymidine family member.

We further provide a cell culture medium comprising a thymidine family member in which a mammalian cell displays enhanced growth or viability compared to a cell culture medium lacking a thymidine family member.

We describe a cell culture medium comprising Formulation A or Formulation B as set out in the Examples.

The animal cell is a Chinese Hamster Ovary (CHO) cell, preferably a suspension-adapted CHO cell. Preferably, the cell culture medium is serum free, and more preferably it is a chemically defined (CD) medium. In such embodiments, the medium comprises a semi-solid medium, preferably, a methylcellulose medium. Preferably, the cell culture medium comprises thymidine and optionally uridine. Preferably, adenosine is present at a reduced or low level, and is preferably absent from the cell culture medium.

The improved cell culture medium has a number of advantages. It enables larger colonies to be obtained in a shorter time than conventional serum free media. Particularly where an automated picking system is used, this enables colonies to be selected at an earlier stage in the growth process. Furthermore, colonies secreting a protein of interest can be assayed more quickly. Finally, the survival of cells is increased.

In particular embodiments, we disclose an animal-free, chemically defined growth supplement for CHO cells in semisolid culture comprising a thymidine family member. The supplement, when added to a serum-free semisolid media for CHO, improves cell viability and proliferation.

Suspension-adapted, serum-free CHO cells benefit from the improved cell culture medium when grown in grown in semisolid media. Cell proliferation is enhanced, which results in larger colonies earlier after plating. Shape and integrity of the resulting colonies can be affected by the surface properties of the culture vessels used. Sterile culture dishes not subjected to surface treatments benefit from virtually background-free growth of suspended CHO colonies suitable for picking within ca. 4 days of incubation.

The presence or increased concentration of thymidine family member in the improved cell culture medium substitutes partially for serum. Thus, in some embodiments, the presence or increased concentration of the thymidine family member may result in the need for less serum in the medium. Thus, it may be preferred to include some serum in the medium, for example 5% or less. The reduction of serum in the medium may be advantageous in reducing the background for protein purification.

In other embodiments, the improved cell culture medium supports the growth of animal cells at a rate or efficiency which is substantially similar to, or better than, an identical cell culture medium comprising serum (but without the presence of or increased concentration of thymidine family member). Preferably, this is the case when the media are formulated as semi-solid media.

The improved cell culture medium is particularly suitable for growing cells for recombinant protein expression. Thus, animal cells may be transfected with expression constructs and plated out for growth. Fast-growing or productive colonies (i.e., those that express or secrete high amount of the protein of interest) may be picked and subcloned preferably by use of a colony picking robot. The cell culture medium is also suitably used for growth of stem cells

A cell or colony of cells which is grown on the surface of or within solid or semi-solid media comprising the improved cell culture medium. Thus, preferably, the cell or colony may be grown on agar, agarose, or methylcellulose media comprising the improved cell culture medium described here. In preferred embodiments, the cells or colonies are grown on a Petri dish or other similar container, although it will be appreciated that other containers may also be used, such as well plates, particularly 1 well plates, 4 well plates, 6 well plates, microtitre dishes, etc.

### Elevated Concentration

By "elevated concentration" or "increased concentration" of a thymidine family member, we mean a concentration of the thymidine family member which is sufficient to increase the rate of growth, or the viability, of a mammalian cell, compared to a cell culture medium which does not comprise the elevated concentration of the thymidine family member. This term can include the mere presence of the thymidine family member in the medium, or preferably an elevated concentration therein.

The thymidine family member may typically be at an elevated or increased concentration relative to a typical minimal essential medium, to which serum would normally be added to form a medium for culture of the animal cell. The thymidine family member may be at an elevated or increased concentration relative to the minimal essential medium, or the minimal essential medium with serum.

The thymidine family member is typically present at a concentration of between about 50 µg/l to about 50 mg/l, preferably between 0.1 mg/l to about 10 mg/l, most preferably between about 0.2 mg/l to about 7 mg/l.

In specific embodiments comprising thymidine, this is preferably present between about 50 µg/l to about 0.5 mg/l, more preferably between 0.1 mg/l to about 0.3 mg/l, more preferably about 0.24 mg/l.

In embodiments comprising uridine, this is preferably present between about 1 mg/l to about 10 mg/l, more preferably between 5 mg/l to about 8 mg/l, more preferably about 7 mg/l.

Preferably, we provide a cell culture medium comprising thymidine at 0.24 mg/L and uridine at 7 mg/l.

### Concentrate or Supplement

The thymidine family member may preferably be provided as a supplement or concentrate, which is added to the basal medium to achieve the higher concentration of the thymidine family member. The supplement or concentrate may for example be provided as a 2x, 5x, 10x, 20x, 50x or 100x supplement.

The concentrations of the components will be increased by the requisite factor, i.e., for a 50X supplement, this may comprise 2.5 mg/l to about 2500mg/l, preferably between 5 mg/l to about 500 mg/l, most preferably between about 10 mg/l to about 350 mg/l of the thymidine family member.

In specific embodiments of a 50x concentrate comprising thymidine, this is preferably present between about 2.5 mg/l to about 25 mg/l, more preferably between 5 mg/l to about 15 mg/l, more preferably about 12 mg/l.

In embodiments of a 50x concentrate comprising uridine, this is preferably present between about 50 mg/l to about 500 mg/l, more preferably between 250 mg/l to about 400 mg/l, more preferably about 350 mg/l.

Preferably, we provide a 50X supplement or concentrate comprising thymidine 12.0 mg/L and uridine at 350.0 mg/l.

### Formulation A

In preferred embodiments, the improved cell culture medium comprises the following:

Calcium Chloride anhydrous (200.00), Potassium Chloride (330.00), Magnesium Sulphate anhydrous (200.00), Sodium Chloride (45405.00), Sodium Dihydrogen Phosphate · H2O (125.00), Sodium Bicarbonate (3024.00), L-Arginine · HCl (84.00), L-Cysteine (48.00), Glycine (30.00), L-Histidine · HCl · H2O (42.00), L-Isoleucine (105.00 ), L-Leucine (105.00), L-Lysine · HCl (146.00), L-Methionine (30.00), L-Phenylalanine (66.00), L-Serine (42.00), L-Threonine (95.00), L-Tryptophan (16.00), L-Tyrosine (72.00), L-Valine (94.00), D-Calcium-Pantothenic (4.00), Choline Chloride (4.00), Folic Acid (4.00), myo-Inositol (7.20), Nicotinamide (4.00), Pyridoxal · HCl (4.00), Riboflavin (0.40), Thiamine · HCl (4.00), D-Glucose anhydrous (4500.0), Sodium Pyruvate (110.00), HEPES (5958.00), L-Alanine (25.00), L-Asparagine H2O (28.40), L-Aspartic Acid (30.00), L-Glutamic acid (75.00), L-Tyrosine disodium salt (104.20), Biotin (0.013), Vitamin B 12 (0.013), Sodium selenite (0.017), L praline (50.00), Ascorbic acid (25.00), Lipoic acid (0.20), Methyl linoleate (0.088), FeSO4 (1.00), ZnSO4 (1.00), CuSO4 (0.0025), Ferric citrate (2.00), supplemented with 8mM glutamine (GlutaMAX™-I, Cat. No. Invitrogen, 35050-038).

### Formulation B

In other embodiments, the improved cell culture medium comprises Formulation B, i.e.:

Calcium Chloride anhydrous (200.00), Potassium Chloride (330), Magnesium Sulphate anhydrous (200.00), Sodium Chloride (45405), Sodium Dihydrogen Phosphate H2O (125.00), Sodium Bicarbonate (3024.00), L-Arginine HCl (84.00), L-Cysteine (48.00), Glycine (30.00), L-Histidine · HCl · H2O (42.00), L-Isoleucine (105.00), L-Leucine (105.00), L-Lysine · HCl (146.00), L-Methionine (30.00), L-Phenylalanine (66.00), L-Serine (52.00), L-Threonine (95.00), L-Tryptophan (16.00), L-Tyrosine (72.00), L-Valine (94.00), D-Calcium-Pantothenic (4.00), Choline Chloride (4.00), Folic Acid (4.00), myo-Inositol (7.20), Nicotinamide (4.00), Pyridoxal · HCl (4.00), Riboflavin (0.40), Thiamine · HCl (4.00), D-Glucose anhydrous (4500.0), Sodium Pyruvate (110.00), HEPES (5958.00), L-Alanine (34.00), L-Asparagine H2O (113.62), L-Aspartic Acid (43.00), L-Glutamic acid (150.00), L-Tyrosine disodium salt (104.20), Biotin (0.013), Vitamin B 12 (0.013), Sodium selenite (0.017), L praline (66.50), Ascorbic acid (25.00), Lipoic acid (0.20), Methyl linoleate (0.088), FeSO4 (1.00), ZnSO4 (1.00), CuSO4 (0.0025), Ferric citrate (2.00), Adenosine (7.00), Guanosine (7.00), Cytidine (7.00), supplemented with 8mM glutamine (GlutaMAX™-I, Cat. No. Invitrogen, 35050-038).

### ANIMAL CELL

The improved cell culture medium is suitable for the culture of any cell or colony or cell line preferably of animal origin. Thus, it will be understood that where reference to culture of a cell is made in this document, this should be taken to include reference to an individual cell, a colony of cells, a cell line, or a tissue comprising a cell.

The animal cell may comprise any animal cultured cell or cell line, as known in the art. Included are established or immortalised cell lines as well as primary cell lines.

A list of known cell lines is set out in the American Type Culture Collection (ATCC, Virginia, USA), Cell Line Data Base (Istituto Nazionale per la Ricerca sul Cancro, Genova, Italy) and the ECACC European Collection of Cell Cultures. Specific examples of cells include CHO cells, HeLa cells, HEK cells, HEK 293 cells, COS 1 cells, embryonic stem cells, African green monkey cells, etc. Such cells may be transfected with suitable expression vectors to enable expression of polypeptides, as described in further detail below. Other cells particularly suitable for use in the methods described here are fused cell lines, including hybridoma cell lines.

Preferably, the animal cell comprises a cell or cell line which is, or has been, adapted to grow in liquid culture, or a suspension-adapted cell or cell line.

In some embodiments, the animal cell comprises a mammalian cell. Preferably, the mammalian cell comprises a rodent cell. In advantageous embodiments, the mammalian cell comprises a hamster cell, preferably a Chinese Hamster *(Cricetulus griseus),* preferably a Chinese Hamster Ovary (CHO) cell.

In one embodiment, the CHO cell comprises a non glutamine dependent CHO cell. In another embodiment, the CHO cell comprises a glutamine dependent CHO cell.

Suitable CHO cell lines for use in the methods and compositions described here are set out in the table below. These cells may be obtained from the depository institution listed, by reference to the deposit numbers set out. In addition, suitable cells and cell lines are available commercially from a number of sources.

| **CHINESE HAMSTER OVARY CELL LINE DESIGNATION** | **DEPOSITORY INSTITUTION AND ACCESSION NUMBER** |
|---|---|
| CHO | ECACC 85050302; ICLC ATL95003 |
| CHO K1 | IZSBS BS CL 15 |
| CHO-DHFR | DSMZ ACC 126; ATCC CCL 61; ECACC 85051005; IZSBS BS CL15; DSMZ ACC 110;ICLC ATL98003 |
| CHO-K1/SF | ECACC 93061607 |
| CHO-SSR1 | ICLC ATL98001 |
| CHO-SSR2 | ICLC ATL98002 |
| CHO/dhFr- | ECACC 94060607 |
| CHO10PV; CHO12RO; CHO181PV; CHO191PV; CHO192PV; CHO201PV; CHO202PV; CHO203PV; CHO204PV; CHO205PV; CHO211PV; CHO23PV; CHO2PV; CHO302PV; CHO30PV; CHO33RO; CHO3PV; CHO40PV; CHO421PV; CHO423PV; CHO43RO; CHO4PV; CHO50PV; CHO51PV; CHO5PV; CHO60PV; CHO7PV; CHO9PV | Available from Istituto Genetica Biochimica ed Evoluzionistica, CNR (PVCGU, Pavia), Italy |
| A2 | ECACC 89030316 |
| A2H | ECACC 85011441 |
| M1WT3 | ECACC 940822109 |
| DXB-11 | Available from Russian Academy of Sciences, SPBIC, St. Petersburg |
| GRL101 (KC7) | ECACC 94071259 |
| GRL101 (MIX) | ECACC 94071258 |
| RR-CHOKI | ECACC 92052129 |
| XrS6 | ECACC 92052128 |
| XrS6-hamKu80 | ECACC 96012301 |
| P22 | ECACC 89030317 |

In advantageous embodiments, the CHO cell line comprises a CHO-S cell line, catalogue number 11619-012, obtained from Gibco.

### ANIMAL CELL CULTURE

Protocols for the culture of animal cells are known in the art, and will generally depend on the nature of the cell and on the purpose for which it will be used. Such protocols are set out in, for example, Ausabel et al, and may be obtained from commercial sources of cell lines.

In general, such protocols may be used for culture of the animal cell, with the replacement of the usual medium with the improved cell culture medium described here, without extensive modification.

It may be necessary however to take into account the faster rate of growth of the animal cell in the replacement medium, and the changes necessary will be evident to the skilled person. Thus, cells may reach stationary phase or confluence sooner, and accordingly cells may need to be passaged earlier in the improved cell culture medium.

Animal cell cultures which have been growing in serum containing medium may need to be adapted gradually to the cell culture medium described here, particularly if it does not contain serum. This may be achieved by subculturing for a number of passages (preferably at least 3), preferably in medium comprising a mix of the original serum containing medium and the serum free medium.

### The following is an example protocol for culture of Chinese Hamster Ovary cells:

Cultures may be grown in shake flasks comprising the cell culture medium described here, with 35 ml cell suspension in a 125 ml shake flask on an orbital shaker platform rotating at 125-135 rpm. Alternatively, or in addition, cultures may be grown in spinner flasks. Cultures are maintained at 37 degrees C +/- 0.5 degrees, in a humidified atmosphere of 5-10% CO₂ in air. The caps of the flasks should be loosened to allow oxygenation or aeration. Cultures may be scaled up as necessary to large volume bioreactors or industrial scale fermentors using methods known to the skilled person-.

### INCREASED CELL GROWTH

The improved cell culture medium described here is capable of maintaining or increasing the growth rate of an animal cell growing in the medium. Thus, an animal cells growing in the improved cell culture medium described here may comprise more cells, and/or proliferate more, and/or have a shorter cell cycle time, and/or have a more rapid increase in cell population, compared to a control cells. The cells may have a higher density.

"Control cells" refers to cognate cells growing in a culture medium without an elevated concentration of the thymidine family member. Such a medium may be referred to as a "control cell culture medium", and will generally comprise a similar composition to the improved cell culture medium, but with a lower concentration of the thymidine family member, or preferably be absent of the thymidine family member.

Preferably, the number of animal cells growing in the improved cell culture medium is more than the number of animal cells in a control population (i.e., growing in a medium without enhanced concentration of thymidine family member). Preferably, the number of animal cells is at least 5%, preferably 10% or more, more preferably 15%, 20%, 30%, 40%, 50% or more than cells in a control population. More preferably, the number of cells is increased by 1x, 2x, 3x, 4x, 5x, 6x, 7x, 8x, 9x, 10x or more compared to the control population.

Preferably, cell growth is assayed by measuring the volume of a colony in a semi-solid medium, using the volume method described below. Preferably, the volume of a colony growing in the improved cell culture medium is at least 2 times the volume of a control colony, preferably 4 times, more preferably 6 times, most preferably 7 times or more.

### Assay for Cell Growth

Cell growth may be assayed by a number of ways, which will be familiar to the person skilled in the art. Cell counts or volume determinations are typically conducted 1, 2, 3, 4, 5, preferably 6 days post splitting or passaging.

One method, which is particularly suitable for suspension cultures, involves determining the cell density by taking a sample volume of culture medium and counting the number of cells in that volume. Counting may be done in a haemocytometer or electronically with a Coulter Counter. Another method plots the number of cells against time; the slope of the graph is steeper for cultures showing improved growth rates.

Another method which is suitable for cells growing in semi-solid medium involves determining the volume of a cell colony ("volume method"). The colony volume may be obtained in a number of ways. For example, an image (such as a photograph) may be taken of the field in a light microscope, and the diameter of the colony measured. the diameter of a single cell is also taken. The volume is calculated by using the formula v=(Π*d3)/6. The colony volume is suitably expressed as a multiple of a cell volume.

A further method which is suitable for assaying cell growth is to provide a count of the number of cells in a colony. This may be done, for example, by counting the cells visible in a colony. Such cell counts may also be used to provide an indication of the number of cell divisions that have taken place since plating. These may be classed in a number of ways; for example, "large" colonies are deemed to be those that have had 10 or more divisions, etc.

The process of assaying cell growth may be automated by use of a robotic colony picker for example. The robotic colony picker comprises imaging means which is capable of capturing a field comprising cells and colonies. Software linked with the mammalian colony picker generates readouts of the average diameter, the area and a measure of roundness for all colonies detected. Use of such automated colony pickers and software enables more accurate data from a large number of colonies analysed.

### INCREASED CELL VIABILITY

The improved cell culture medium described here is capable of maintaining or increasing the cell viability of an animal cell growing in the medium. Thus, the animal cells may survive in culture longer, in terms of time or number of generations.

Preferably, cell viability is gauged by quantitating a viable cell density of a population of cells growing in the cell culture medium. Preferably, the animal cells maintain a higher cell viability, compared to cells growing in medium which does not contain elevated thymidine family member (e.g., a control population). Cell viability is preferably measured as the percentage of cells in the relevant cell population which are viable.

Preferably, the animal cells growing in the improved cell culture medium have at least 5%, preferably 10% or more, more preferably 15%, 20%, 30%, 40%, 50% or more viable cells compared to a control population. Alternatively, or in addition, such cells maintain cell viability for a longer period of time compared to cells of the control population. For example, cells growing in the improved culture medium described here are able to maintain a certain percentage cell viability (e.g., 95%) for a longer period compared to control cells.

Preferably, cells grown in the improved cell culture medium have extended cell viability by at least 1 hour, more preferably at least 6 hours, most preferably at least 12 hours or more, e.g., at least 24 hours, at least 36 hours or at least 48 hours, compared to control cells. In highly preferred embodiments, such cells have extended viability by at least 24 hours before viability begins to drop below 95%, compared to control cells.

Cells growing in the improved cell culture medium described here preferably are capable of higher viable culture densities compared to control cells. Preferably, such cells are capable of 10%, 20%, 30%, 40%, 50%, 60%, 70%, 80%, 90%, 100%, 150%, 200% or higher viable cell density compared to control cells. For example, cells may achieve densities as high as 9.6 x 10⁶ cells / ml when growing or grown in the improved cell culture medium.

Cells growing in the improved cell culture medium preferably display a delayed onset of expression of an apoptosis marker, preferably caspase 2, caspase 3 or caspase 8. Such cells may have the property of displaying reduced apoptosis, in terms of longer time of survival for individual cells, or the number of cells which display apoptosis. Preferably, they have the property of being resistant to apoptosis (see below).

### Assay for Cell Viability

In a preferred embodiment, cell viability is determined by a "Trypan blue viability exclusion assay". This assay is commonly used for cell viability determination in the field of cell culture. A detailed protocol comprises the following: a cell suspension is mixed with 0.4% trypan blue in phosphate buffered solution and counted using a hemocytometer. Live cells appear round and refractile without any blue-dye coloration while dead cells absorb the dye and appear blue. Viability is then expressed as a percentage of viable cells over total cells counted.

A viable cell is defined as a cell that whose membrane integrity is still able to prevent the absorption of trypan blue in a trypan blue exclusion viability assay.

### INCREASED PROTEIN YIELD

Advantageously, animal cells when growing or grown in the improved cell culture medium described here are capable of increased protein yield, preferably increased recombinant expressed protein yield, compared to control cells.

Preferably, such cells are capable of 10%, 20%, 30%, 40%, 50%, 60%, 70%, 80%, 90%, 100%, 150%, 200% or more higher yield compared to control cells. More preferably, such cells are capable of 2.5x, 3x, 5x, 10x or more higher yield compared to control cells. We therefore provide a method of expressing a recombinant protein, preferably a biotherapeutic molecule, in an improved cell culture medium as described herein.

Preferably, such cells display any one or more of their properties in batch culture, fed-batch culture or preferably both.

Identification of suitable colonies growing in the improved cell culture medium, which colonies have increased productivity (i.e., able to secrete or express more protein) may for example be conducted by the use of a class marker and a specificity marker, as described in US utility application 11/199,243, herein incorporated by reference.

### OTHER FEATURES

The use of the improved cell culture medium may further result in other advantages.

### Structural and/or Positional Integrity of Colonies

In advantageous embodiments, use of the improved cell culture medium results in the formation of colonies of high structural and positional integrity, particularly in semi-solid media. In other words, the colonies are not easily broken up, and are stable during manipulation or movement of containers housing them. This may be as a result of increased cohesiveness between cells and/or reduced adhesiveness between cells and the substrate or a wall of the container.

Furthermore, the colonies maintain their position with respect to the container and with respect to other colonies. These two advantages are highly beneficial for manual as well as automated colony picking.

### Transplantation Shock

In an advantageous embodiment, the use of the improved cell culture medium is capable of reducing the stress on tissue culture cells growing in the medium, for example, when manipulated using laboratory techniques. Examples of manipulation include: passaging, splitting, removal from substrate, exposure to protease, exposure to proteinase, centrifugation, washing, etc. Thus, for example, during the process of passaging or splitting a culture, there is typically a delay or lag between exposing cells to the fresh medium and the cells starting to grow. Thus, when cells are split and plated on a medium, for example, it will take some time before they start entering the cell cycle and dividing. The use of the improved cell culture medium reduces by 10%, 20%, 30%, 40%, 70%, 90% or 100% such stress or delay or abolishes such stress or delay.

In a specific embodiment, the use of the improved cell culture medium reduces "transplantation shock", which is the shock or stress experienced when a cell or colony is transferred from one medium to another, including splitting and passaging. The starting medium and the ending medium may be of the same type (e.g., semi-solid media to semi-solid media, liquid media to liquid media) or may be of different types (e.g., semi-solid media to liquid media, or *vice versa*).

### Plating Efficiency

When cells are plated out, typically not all the cells are able to form colonies. In fact, typically only a very small percentage do. Thus, we find that a typical "plating efficiency" is in the order of less than 0.1 % (see the Examples).

The plating efficiency is defined as the number of colonies actually formed, over the number of cells plated out. In another advantageous embodiment, therefore, the use of the improved cell culture medium is capable of increasing the plating efficiency of cultured animal cells.

In specific embodiments, use of our improved cell culture medium can give rise to plating efficiencies of up to 1% or more. The improved cell culture medium may be present in the starting medium or the final medium or both.

In a preferred embodiment, the plating efficiency is increased by 2x, 5x, 10, 20, 25x or more, compared to use of a control medium.

### APPLICATIONS

The improved cell culture medium described here may be used in a number of situations.

For example, in the production of monoclonal antibodies, an essential step is hybridoma selection, including the separation and culture of individual hybridoma clones (fused myelomas and primary mouse cells). After cell fusion, the traditional way of selecting for monoclonality is to plate out single cells into 96-well dishes. This is repeated until clonality is assured. Use of the improved cell culture medium allows faster growth of plated out cells and a reduction of time in achieving mono-clonality.

Similarly, understanding gene function and identification of pharmaceutical leads requires the establishment of cell lines containing transfected genes expressed at an appropriate level. Standard techniques require the co-transfection of a gene with a dominant selectable marker followed by selection for growth for example in an antibiotic such as G418 or hygromycin. The resulting colonies are then picked by hand and further analysed for gene expression (RT-PCR) and functional expression. Growth of the colonies in the improved cell culture medium allows faster growth and a reduction of time in locating clones expressing the correct protein or productive clones.

Ascertaining optimal conditions for stem cell growth and differentiation requires broad testing of growth factors and culture conditions. The evaluation of a particular treatment requires a statistical approach on a large number of individual cells. One way to achieve this is to use numerous culture dishes, several for each treatment. Use of the improved cell culture medium allows faster growth of stem cells.

This process of cloning out may be modified and automated through the use of robots. Thus, for example, the ClonePix robot (manufactured by Genetix) implements this process by picking individual colonies directly from standard semi-solid media, the media preventing migration of the dividing cells. Thus, an imaging head captures images of colonies growing in the medium under white light, and software routines allow the separation and detection of individual colonies. A picking head then picks individual colonies into a 96-well plate.

One advantage of a robot implemented picking method is that colonies grown for a short time can be picked into 96-well plates. The picking speed can be up to 400 clones per hour and graphic software allows the user to select colonies on the basis of size, shape, brightness and proximity. Furthermore, the software allows stratification of clones into slow, medium and fast growing cells, and clones of the same class may be grouped in the same 96-well plate. This gives rise to considerable savings in subsequent tissue culture steps as all wells can be processed at the same time.

The use of the improved cell culture medium in conjunction with a robot implemented picking method enables further efficiencies to be achieved, due to the further shortening of time needed for colonies to grow to a size which enables them to be picked.

### SOLID OR SEMI-SOLID MEDIA

In preferred embodiments, the cell culture medium comprises a solid or semi-solid medium. According to this embodiment, we provide a solid or semi-solid cell culture medium comprising an elevated concentration of a thymidine family member. Preferably, such a medium is serum-free.

For the purposes of this document, a "semi-solid medium" should be understood as a medium having a gel-like consistency or viscosity. Semi-solid media may be optimized to support the growth and proliferation of animal cells such as CHO cells forming discreet colonies. Preferably, such cell colonies maintain their integrity and position within the culture dish enabling such colonies to be monitored, picked and/ or subsequently characterised.".

Growth of cells, particularly antibody secreting hybridomas, on such media enhances secretion, as described in Goding, J.W. 1980. Antibody production by hybridomas. [Review]. J. Immunol. Methods. 39(4): 285-308, Sharon, J., Morrison, S.L. and Kabat, E.A. 1979. Detection of specific hybridoma clones by replica immunoadsorption of their secreted antibodies. Proc. Natl. Acad. Sci. (USA). 76(3): 1420-4 and Davis, J.M., Pennington, J.E., Kubler, A.-M. and Conscience, J.F. 1982. A simple, single-step technique for selecting and cloning hybridomas for the production of monoclonal antibodies. J. Immunol. Methods. 50: 161-171.

Methylcellulose media may be formulated at any suitable concentration, for example, 0.5% to 3%, preferably 1% or 2%.

Methylcellulose media may, for example, be obtained from Sigma-Aldrich Company Ltd (Dorset, UK) under catalogue number M0387 (Methyl cellulose viscosity 1,500 cP (2% aqueous solution, 20 °C) (lit.) CAS Number 9004-67-5) or catalogue number M0512 (Methyl cellulose viscosity 4,000 cP (2% aqueous solution, 20 °C) (lit.) CAS Number 9004-67-5). A preferred methylcellulose media comprises CloneMatrix and CloneMatrix-CHO (Genetix, New Milton, United Kingdom).

In highly preferred embodiments, the animal cell or colony of cells is grown on the surface of or within such methylcellulose media. The use of methylcellulose media is well known in the art, and protocols have been established to enable hybridoma cloning on such media. See for example, the ClonaCell™ - HY Hybridoma Cloning Kit Procedure Manual (StemCell Technologies, Vancouver, Canada), herein incorporated by reference.

The media may optionally comprise growth factors or other supplements optimized to support the selection and growth of the relevant cells.

### CELL CULTURE MEDIUM

To prepare the improved cell culture medium, the thymidine family member, preferably thymidine and/or uridine may be added to a commercially available culture medium for culture of an animal cell. For this purpose, the thymidine family member may be formulated and provided as a supplement or concentrate, for example as a 2x, 5x, 10x, 20x, 50x or 100x supplement.

In some embodiments, the cell culture medium may comprise some volume of conditioned medium. Conditioned medium refers to medium in which the animal cells have been growing for at least some time. Preferably, the conditioned medium is obtained from the culture supernatant. The conditioned medium may be sterile-filtered through a 2um pore-sized syringe filter. The conditioned medium may be derived from the supernatant day 2 post-passaging

In such embodiments, preferably the improved cell culture medium comprises between 0.01x to 0.5x of conditioned medium, preferably between 0.05x to 0.3x, more preferably between 0.1x to 0.2x, most preferably 0.2x.

Suitable media for supplementation include, for example, BME medium, MEM medium, DMEM medium, F10 medium, or F12 medium. Thus, commercially available chemically-defined CHO media (e.g. GIBCO CD CHO medium, Invitrogen, Cat.No. 10743-011 or GIBCO CD CHO AGT granular medium, Invitrogen, Cat. No. 12490-017 or EX-CELL™ 302 Serum-Free Medium for CHO Cells, JRH 14324-500M or EX-CELL™ 302 Serum-Free Medium for CHO Cells, Dry Powder Medium (DPM), JRH, 24324-1L) may be used. In preferred embodiments, the basal medium comprises Gibco CD CHO Medium.

The improved cell culture medium may comprise a portion of "conditioned medium", preferably between 5% to 30% of total volume. The conditioned medium comprises medium in which the cells have been growing, preferably for a period. Preferably, the conditioned medium is the medium obtained immediately or soon after splitting or passaging of cells, preferably 2 days post-splitting. The conditioned medium may be sterilised , preferably by filtration.

As other components of the culture medium, various components usually used in animal cell culture media can be used as desired. They include amino acids, vitamins, lipid factors, energy sources, osmotic regulators, iron sources, and pH regulators. In addition to these components, trace metal elements, surfactants, growth cofactors, and nucleosides may be added.

For example, amino acids, such as L-alanine; L-cystine; L-proline; L-tyrosine; L-arginine; L-histidine; L-leucine; L-lysine; L-serine; L-valine; L-methionine; L-ornithine; L-phenylalanine; L-glutamic acid; glycine; L-tryptophan; L-asparagine; L-glutamine; L-aspartic acid; L-cysteine; L-isoleucine and L-threonine may be added. Preferably the amino acid comprises L-alanine; L-aspartic acid; L-cystine; L-arginine; L-leucine; L-glutamic acid; L-histidine; L-isoleucine; L-serine; L-lysine; L-tryptophan; L-tyrosine; glycine; L-methionine; L-phenylalanine; L-proline; L-asparagine; L-glutamine; L-threonine; and L-valine.

Other components may include lipid factors, such as choline tartrate, linoleic acid, oleic acid, choline chloride and cholesterol, preferably choline chloride. Vitamins, such as nicotinamide; nicotinic acid; vitamin B 12; ascorbic acid; pyridoxal hydrochloride; pyridoxine hydrochloride; riboflavin; i-inositol; biotin; p-aminobenzoic acid; calcium pantothenate; thiamine hydrochloride; folic acid; and lipoic acid may be included. Preferably, the vitamin comprises riboflavin; pyridoxal hydrochloride; biotin; nicotinamide; calcium pantothenate; thiamine hydrochloride; vitamin B 12; ascorbic acid; folic acid; or lipoic acid.

Also included may be iron sources, such as iron EDTA, ferric citrate, ferrous chloride, ferric chloride, ferrous sulfate, ferric sulfate, and ferric nitrate, preferably, ferric chloride, iron EDTA, and ferric citrate; osmotic regulators, such as sodium chloride, potassium chloride, and potassium nitrate, preferably, sodium chloride; energy sources, such as glucose, galactose, mannose, and fructose, preferably, glucose; and pH regulators, such as sodium bicarbonate, calcium chloride, sodium phosphate monobasic, HEPES, and MOPS, preferably, sodium bicarbonate. Culture media containing any of these components can be cited as examples.

Besides the above components, there may be added trace metal elements, such as copper sulfate, manganese sulfate, zinc sulfate, magnesium sulfate, nickel chloride, tin chloride, magnesium chloride, and sodium subsilicate, preferably, copper sulfate, zinc sulfate, and magnesium sulfate; surfactants, such as Tween 80, and Pluronic F68; growth cofactors, such as recombinant insulin, recombinant IGF, recombinant EGF, recombinant FGF, recombinant PDGF, recombinant TGF-α, ethanolamine hydrochloride, sodium selenite, retinoic acid, and putrescine dihydrochloride, preferably, sodium selenite, ethanolamine hydrochloride, recombinant IGF, and putrescine dihydrochloride; and nucleosides, such as deoxyadenosine, deoxycytidine, deoxyguanosine, adenosine, cytidine, guanosine, and uridine.

In preferred embodiments, antibiotics, such as streptomycin, penicillin-G potassium, and gentamicin, and pH-indicators, such as Phenol Red, may be contained.

The amounts of the other components in the culture medium may be 0.05 to 1,500 mg/L for amino acids, 0.001 to 10 mg/L for vitamins, 0 to 200 mg/L for lipid factors, 1 to 20 g/L for energy sources, 0.1 to 10,000 mg/L for osmotic regulators, 0.1 to 500 mg/L for iron sources, 1 to 10,000 mg/L for pH buffers, 0.00001 to 200 mg/L for trace metal elements, 0 to 5,000 mg/L for surfactants, 0.05 to 10,000 µg/L for growth cofactors, and 0.001 to 50 mg/L for nucleosides. Their amounts can be determined, as required, according to the type of the animal cell to be cultured, and the type of the desired protein to be produced.

The pH of the culture medium differs according to the cell to be cultured, but is generally pH 6.8 to 7.6, or pH 7.2 to 7.4 in many cases.

The culture medium can be used, without any restriction, for preferably culturing various animal cells. For example, there can be cultured a COS cell or CHO cell having a gene for a protein of interest, for example a desired antibody or physiologically active substance incorporated by a genetic engineering procedure, or an antibody-producing fused cell typified by a hybridoma, such as mouse-human, mouse-mouse, or mouse-rat. The improved culture medium is particularly preferred for culture of a CHO cell. Needless to say, the culture medium for culture of an animal cell can be used when culturing an animal cell to obtain a natural protein produced by the animal cell. The culture medium can be used for culture of a BHK cell and a HeLa cell as well as the above-mentioned cells.

The culture conditions differ according to the type of the cell used, and preferred conditions may be determined as desired. A CHO cell, for example, is usually cultured for 1 to 14 days in an atmosphere with a CO2 concentration in a gas phase of 0 to 40%, preferably 2 to 10%, at 30 to 39° C., preferably about 37° C.

Culture can be performed using various culture devices for animal cell culture, for example, a fermentor type tank culture device, an air lift type culture device, a culture flask type culture device, a spinner flask type culture device, a microcarrier type culture device, a fluidized bed type culture device, a hollow fiber type culture device, a roller bottle type culture device, and a packed bed type culture device.

By performing culture in the culture medium for culture of an animal cell, a protein produced by the animal cell can be obtained in the culture medium. To produce the protein from the animal cell, mere culture may suffice, or a special procedure may be required. The procedure, conditions, etc. may be determined, as required, according to the animal cell to be cultured. In the case of a CHO cell transformed with a vector containing a gene encoding a mouse-human chimeric antibody by a genetic engineering operation, for example, culture is performed under the aforementioned conditions, whereby the desired protein can be obtained in the culture medium in about 1 to 14 days, preferably in about 7 to 10 days. Then, the culture medium is subjected to isolation and purification by customary methods (see, for example, Introduction to Antibody Engineering, Chijin Sho Kan publishing company, pp. 102-104; Affinity Chromatography Principles & Methods, Amersham Pharmacia Bitech, pp. 56-60), whereby the desired protein can be obtained.

The foregoing methods for production can produce gene recombinant proteins, such as recombinant antibodies such as anti-human IL-6 receptor antibody (including chimeric antibodies, humanized antibodies, human antibodies), granulocyte colony stimulating factor (G-CSF), granulocyte macrophage colony stimulating factor (GM-CSF), erythropoietin, interferon, interleukins (e.g., IL-1 and IL-6), t-PA, urokinase, serum albumin, and blood coagulation factor VIII.

### APOPTOSIS

Animal cells cultured in the improved cell culture medium described here display improved cell viability. In preferred embodiments, the improved cell viability results from a reduction of apoptosis in the animal cells.

Such animal cells are preferably capable of maintaining a higher viable cell density, preferably for a longer period of time, compared to control cells. Preferably, the number of viable cells in a population growing in the improved cell culture medium is higher, for example 10%, 20%, 30%, 40%, 50%, 100%, 200%, 500%, or more, compared to an control population growing in a cell culture medium without elevated concentrations of the thymidine family member.

Preferably, the cells show an extension of viability by at least 6 hours, at least 12 hours, preferably at least 18 hours, and most preferably at least 24 hours compared to control cells. In highly preferred embodiments, caspase 2 and / or caspase 3 and/or caspase 8 expression is delayed by such times compared to control cells.

Accordingly, preferably, apoptosis in a cell population growing in the improved cell culture medium is decreased by at least 10%, preferably 25% or more, more preferably 40%, 50%, 75%, 95% or more compared to a control population. In preferred embodiments, the percentage of apoptotic cells in such cell population is decreased by such amounts. In highly preferred embodiments, such cells are resistant to apoptosis, i.e., display little or no significant apoptosis.

Methods of assaying apoptosis are known in the art, and are described in detail below

An alternative assay of apoptosis involves quantitation or measurement of levels any one or more of caspase 2, caspase 3 and caspase 8 in the relevant cells. Thus, preferably, levels of any one or more of these caspases is decreased in a cell or population growing in the improved cell culture medium compared to one growing in a medium without elevated concentrations of thymidine family member, by 10%, 20%, 30%, 50%, 70%, 80%, 90% or more. Preferably, such cells exhibit a delay in expression of any one or more of caspase 2, caspase 3 and caspase 8 by a period of time preferably at least 1 hour, more preferably at least 6 hours, most preferably at least 12 hours or more, e.g., at least 24 hours, at least 36 hours, at least 48 hours, at least 72 hours, at least 144 hours, at least 288 hours, or more, compared to control cells. Caspase levels may be assayed by any means known in the art, including RT-PCR, RNAse protection, SDS-PAGE, immunoassays, etc.

Cell death can occur by either of two distinct mechanisms, necrosis or apoptosis. In addition, certain chemical compounds and cells are said to be cytotoxic to the cell, that is, to cause its death.

"Cytotoxicity" refers to the cell killing property of a chemical compound (such as a food, cosmetic, or pharmaceutical) or a mediator cell (cytotoxic T cell). In contrast to necrosis and apoptosis, the term cytotoxicity need not necessarily indicate a specific cellular death mechanism. For example, cell mediated cytotoxicity (that is, cell death mediated by either cytotoxic T lymphocytes [CTL] or natural killer [NK] cells) combines some aspects of both necrosis and apoptosis.

"Necrosis" (also referred to as "accidental" cell death) refers to the pathological process which occurs when cells are exposed to a serious physical or chemical insult. Necrosis occurs when cells are exposed to extreme variance from physiological conditions (e.g., hypothermia, hypoxia) which may result in damage to the plasma membrane. Under physiological conditions direct damage to the plasma membrane is evoked by agents like complement and lytic viruses. Necrosis begins with an impairment of the cell's ability to maintain homeostasis, leading to an influx of water and extracellular ions. Intracellular organelles, most notably the mitochondria, and the entire cell swell and rupture (cell lysis). Due to the ultimate breakdown of the plasma membrane, the cytoplasmic contents including lysosomal enzymes are released into the extracellular fluid. Therefore, *in vivo,* necrotic cell death is often associated with extensive tissue damage resulting in an intense inflammatory response.

"Apoptosis" ("normal" or "programmed" cell death) refers to the physiological process by which unwanted or useless cells are eliminated during development and other normal biological processes. Apoptosis is a mode of cell death that occurs under normal physiological conditions and the cell is an active participant in its own demise ("cellular suicide"). It is most often found during normal cell turnover and tissue homeostasis, embryogenesis, induction and maintenance of immune tolerance, development of the nervous system and endocrinedependent tissue atrophy. Cells undergoing apoptosis show characteristic morphological and biochemical features. These features include chromatin aggregation, nuclear and cytoplasmic condensation, partition of cytoplasm and nucleus into membrane bound vesicles (apoptotic bodies) which contain ribosomes, morphologically intact mitochondria and nuclear material. *In vivo*, these apoptotic bodies are rapidly recognized and phagocytized by either macrophages or adjacent epithelial cells. Due to this efficient mechanism for the removal of apoptotic cells in vivo no inflammatory response is elicited. *In vitro,* the apoptotic bodies as well as the remaining cell fragments ultimately swell and finally lyse. This terminal phase of in vitro cell death has been termed "secondary necrosis".

Table 1 summarises the various observable differences between necrosis and apoptosis. Preferably, cells growing in improved cell culture medium exhibit a reduction in one or more of these features. Any of these differences, alone or in combination, may be assayed in order to determine whether cell death is occurring by apoptosis or by necrosis.

**Table 1: Differential features and significance of necrosis and apoptosis.**

| | **Necrosis** | **Apoptosis** |
|---|---|---|
| **Morphological features** | Loss of membrane integrity Begins with swellinof cytoplasm and mitochondria Ends with total cell lysis No vesicle formation, complete lysis Disintegration (swelling) of organelles | Membrane blebbing, but no loss of integrity Aggregation of chromatin at the nuclear membrane Begins with shrinking of cytoplasm and condensation of nucleus Ends with fragmentation of cell into smaller bodies Formation of membrane bound vesicles (apoptotic bodies) Mitochondria become leaky due to pore formation involving proteins of the bcl-2 family. |
| **Biochemical features** | Loss of regulation of ion homeostasis No energy requirement (passive process, also occurs at 4°C) Random digestion of DNA (smear of DNA after agarose gel electrophoresis) Postlytic DNA fragmentation (= late event of death) | Tightly regulated process involving activation and enzymatic steps Energy (ATP)-dependent (active process, does not occur at 4°C) Non-random mono- and oligonucleosomal length fragmentation of DNA (Ladder pattern after agarose gel electrophoresis) Prelytic DNA fragmentation Release of various factors (cytochrome C, AIF) into cytoplasm by mitochondria Activation of caspase cascade Alterations in membrane asymmetry (i.e., translocation of phosphatidyl-serine from the cytoplasmic to the extracellular side of the membrane) |
| **Physiological significance** | Affects groups of contiguous cells Evoked by non-physiological disturbances (complement attack, lytic viruses, hypothermia, hypoxia, ischemica, metabolic poisons) Phagocytosis by macrophases Significant inflammatory response | Affects indivual cells Induced by physiological stimuli (lack of growth factors, changes in hormnal environment) Phagocytosis by adjacent cells or macrophases No inflammatory response |

Reference is made to the following documents, which describe apoptosis in detail, as well as various assays for measuring cell death by apoptosis: Schwartzman, R. A. and Cidlowski, J. A. (1993). Endocrine Rev. 14, 133; Vermes, I. and Haanan, C. (1994). Adv. Clin. Chem. 31, 177; Berke, G. (1991). Immunol. Today 12, 396; Krähenbühl, O. and Tschopp, J. (1991). Immunol. Today12, 399; Van Furth, R. and Van Zwet, T. L. (1988). J. Immunol; Methods 108, 45. Cohen, J. J. (1993) Apoptosis. Immunol. Today14, 126; Savill, J. S. et al. (1989). J. Clin. Invest. 83, 865; Wyllie, A. H. (1980). Nature 284, 555; Leist, M. et al. (1994) Biochemica No. 3, 18-20; Fraser, A. and Evan, G. (1996) Cell 85, 781-784; Duke, R. C. (1983). Proc. Natl. Acad. Sci. USA 80,6361; Duke, R. C. & Cohen, J. J. (1986). Lymphokine Res. 5, 289; Trauth, B. C. et al. (1994) Eur. J. Cell. Biol. 63, 32,Suppl 40; Matzinger, P. (1991). J. Immunol; Methods 145, 185; Kaeck, M. R. (1993); Anal. Biochem. 208, 393; Prigent, P. et al. (1993). J. Immunol; Methods 160, 139; Huang, P. & Plunkett, W. (1992); Anal. Biochem. 207, 163Bortner , C. D. et al. (1995) Trends Cell Biol. 5, 21; Gold, R. et al. (1994); Lab. Invest. 71, 219.

Apoptosis and cell mediated cytotoxicity are characterized by cleavage of the genomic DNA into discrete fragments prior to membrane disintegration. Accordingly, apoptosis may be assayed by measuring DNA fragmentation, for example, by observing the presence of DNA ladders. DNA fragments may be assayed , for example, as "ladders" (with the 180 bp multiples as "rungs" of the ladder) derived from populations of cells, or by quantification of histone complexed DNA fragments via, for exmaple, ELISA. Such an assay relies on an one-step sandwich immunoassay to detect nucleosomes. The procedure involves pelleting cells by centrifugation and discarding the supernatant (which contains DNA from necrotic cells that leaked through the membrane during incubation). Cells are resuspended and incubated in lysis buffer. After lysis, intact nuclei are pelleted by centrifugation. An aliquot of the supernatant is transferred to a streptavidin-coated well of a microtiter plate, and nucleosomes in the supernatant are bound with two monoclonal antibodies, anti-histone (biotin-labeled) and anti-DNA (peroxidase-conjugated). Antibody-nu-cleosome complexes are bound to the microtiter plate by the streptavidin. The immobilized antibody-histone complexes are washed three times to remove cell components that are not immuno-reactive, and the sample is incubated with peroxidase sub-strate (ABTS ® ). The amount of colored product (and thus, of immobilized antibody- histone complexes) is then determined spectrophotometrically.

Several proteases are involved in the early stages of apoptosis. Apoptosis may therefore also be assayed by detecting the presence of, in addition to, or instead of, assaying the activity of, apoptosis-induced proteases such as caspases, e.g., caspase 3. Caspase activation can be analyzed in different ways, for example, by an *in vitro* enzyme assay of, for example, cellular lysates by capturing of the caspase and measuring proteolytic cleavage of a suitable substrate. Furthermore, caspases may be assayed by detection of cleavage of an *in vivo* caspase substrate such as PARP (Poly-ADP-Ribose-Polymer-ase). Cleaved fragments of PARP may be detected with a suitable antibody such as an anti PARP antibody. Protease assays and DNA fragmentation assays are especially suitable for assaying apoptosis in cell populations.

Methods for studying apoptosis in individual cells are also available, such as ISNT and TUNEL enzymatic labeling assays. As noted above, extensive DNA degradation is a characteristic event which often occurs in the early stages of apoptosis. Cleavage of the DNA yields double-stranded, low molecular weight DNA fragments (mono- and oligonucleosomes) as well as single strand breaks ("nicks") in high molecular weight-DNA. In TUNEL, such DNA strand breaks are detected by enzymatic labeling of the free 3'-OH termini with suitable modified nucleotides (such as X-dUTP, X = biotin, DIG or fluorescein). Suitable labeling enzymes include DNA polymerase (nick translation) in ISNT *("in situ* nick translation") and terminal deoxynucleotidyl transferase (end labeling) in TUNEL ("TdT-mediated X-dUTP nick end labeling"; Huang, P. & Plunkett, W., 1992, Anal. Biochem. 207, 163; Bortner , C. D. et al., 1995, Trends Cell Biol. 5, 21).

Apoptosis may also be assayed by measuring membrane alterations, including: loss of terminal sialic acid residues from the side chains of cell surface glycoproteins, exposing new sugar residues; emergence of surface glycoproteins that may serve as receptors for macrophage-secreted adhesive molecules such as thrombospondin; and loss of asymmetry in cell membrane phospholipids, altering both the hydrophobicity and charge of the membrane surface. In particular, the human anticoagulant annexin V is a 35-36 kilodalton, Ca2+-dependent phospholipid-binding protein that has a high affinity for phosphatidylserine (PS). In normal viable cells, PS is located on the cytoplasmic surface of the cell membrane. However, in apoptotic cells, PS is translocated from the inner to the outer leaflet of the plasma membrane, thus exposing PS to the external cellular environment. Annexin V may therefore be used to detect phos-phatidylserine asymmetrically exposed on the surface of apoptotic cells (Homburg, C. H. E. et al. 1995, Blood 85, 532; Verhoven, B. et al., 1995, J. Exp. Med. 182, 1597). Furthermore, DNA stains such as DAPI, ethidium bromide and propidium iodide, etc may be used for differential staining to distinguish viable and non-viable cells. Profiles of DNA content may also be used; thus, permeabilized apoptotic cells leak low molecular weight DNA, and detection of "sub-G 1 peaks", or "A 0 " cells (cells with lower DNA staining than that of G 1 cells) may be detected by, for example, flow cytometry. Morphological changes characteristic of apoptosis may also be detected in this manner.

Detection of apoptosis-related proteins such as ced-3, ced-4, ced-9 (Ellis, H. M. and Horvitz, H. R., 1986, Cell 44, 817-829; Yuan, J. Y. and Horvitz, H. R., 1990, Dev. Biol. 138, 33-41; Hentgartner, M. O., Ellis, R. E. and Horvitz, H. R., 1992, Nature 356, 494-499.), Fas(CD95/Apo-1; Enari et al., 1996, Nature 380, 723-726), Bcl-2 (Baffy, G. et al., 1993, J. Biol. Chem. 268, 6511-6519; Miyashita, T. and Reed, J. C., 1993, Blood 81, 151-157; Oltvai, Z. N., Milliman, C. L. and Korsmeyer, S. J., 1993, Cell 74, 609-619), p53 (Yonish-Rouach, E. et al., 1991, Nature 352, 345-347), etc by the use of antibodies may also be used to assay apoptosis.

### PROTEIN EXPRESSION

The improved cell culture medium is suitable for culturing cells for the production of proteins. We therefore provide methods of production of proteins of interest, particularly recombinant proteins, by expression from cells cultured in the improved cell culture medium described here.

Such proteins of interest may comprise recombinant proteins, produced from cells, e.g., CHO cells, and are described in more detail below. It is within the skills of the person skilled in the art to transfect or transform such cells with suitable expression constructs or vectors for the expression of heterologous proteins.

Expression and cloning vectors usually contain a promoter that is recognised by the host organism and is operably linked to a nucleic acid encoding the protein of interest.

Such a promoter may be inducible or constitutive. The promoters are operably linked to DNA encoding the protein of interest by removing the promoter from the source DNA by restriction enzyme digestion and inserting the isolated promoter sequence into the vector. Both the native promoter sequence of the protein of interest and many heterologous promoters may be used to direct amplification and/or expression of DNA encoding the protein of interest.

Promoters suitable for use with prokaryotic hosts include, for example, the β-lactamase and lactose promoter systems, alkaline phosphatase, the tryptophan (Trp) promoter system and hybrid promoters such as the tac promoter. Their nucleotide sequences have been published, thereby enabling the skilled worker operably to ligate them to DNA encoding the protein of interest protein, using linkers or adapters to supply any required restriction sites.

Expression of proteins from animal cells may comprise gene transcription from vectors in mammalian hosts; this may be controlled by promoters derived from the genomes of viruses such as polyoma virus, adenovirus, fowlpox virus, bovine papilloma virus, avian sarcoma virus, cytomegalovirus (CMV), a retrovirus and Simian Virus 40 (SV40), from heterologous mammalian promoters such as the actin promoter or a very strong promoter, e.g. a ribosomal protein promoter, and from the promoter normally associated with the sequence of the gene encoding protein of interest, provided such promoters are compatible with the host cell systems.

Transcription of a DNA encoding a protein of interest by higher eukaryotes may be increased by inserting an enhancer sequence into the vector. Enhancers are relatively orientation and position independent. Many enhancer sequences are known from mammalian genes (e.g. elastase and globin). However, typically one will employ an enhancer from a eukaryotic cell virus. Examples include the SV40 enhancer on the late side of the replication origin (bp 100-270) and the CMV early promoter enhancer. The enhancer may be spliced into the vector at a position 5' or 3' to the coding sequence, but is preferably located at a site 5' from the promoter.

Advantageously, a eukaryotic expression vector encoding a protein of interest may comprise a locus control region (LCR). LCRs are capable of directing high-level integration site independent expression of transgenes integrated into host cell chromatin, which is of importance especially where the protein of interest is to be expressed in the context of a permanently-transfected eukaryotic cell line in which chromosomal integration of the vector has occurred, or in transgenic animals.

Eukaryotic vectors may also contain sequences necessary for the termination of transcription and for stabilising the mRNA. Such sequences are commonly available from the 5' and 3' untranslated regions of eukaryotic or viral DNAs or cDNAs. These regions contain nucleotide segments transcribed as polyadenylated fragments in the untranslated portion of the mRNA encoding the protein of interest.

An expression vector includes any vector capable of expressing a nucleic acid sequence encoding a protein of interest. The expression vector may comprise the coding sequence operatively linked with regulatory sequences, such as promoter regions, that are capable of expression of such DNAs. Thus, an expression vector refers to a recombinant DNA or RNA construct, such as a plasmid, a phage, recombinant virus or other vector, that upon introduction into an appropriate host cell, results in expression of the cloned DNA. Appropriate expression vectors are well known to those with ordinary skill in the art and include those that are replicable in eukaryotic and/or prokaryotic cells and those that remain episomal or those which integrate into the host cell genome. For example, DNAs encoding a protein of interest may be inserted into a vector suitable for expression of cDNAs in mammalian cells, e.g. a CMV enhancer-based vector such as pEVRF (Matthias, et al., (1989) NAR 17, 6418).

Particularly useful are expression vectors that provide for the transient expression of DNA encoding the protein of interest protein in mammalian cells. Transient expression usually involves the use of an expression vector that is able to replicate efficiently in a host cell, such that the host cell accumulates many copies of the expression vector, and, in turn, synthesises high levels of the protein of interest.

Construction of vectors employs conventional ligation techniques. Isolated plasmids or DNA fragments are cleaved, tailored, and religated in the form desired to generate the plasmids required. If desired, analysis to confirm correct sequences in the constructed plasmids is performed in a known fashion. Suitable methods for constructing expression vectors, preparing in vitro transcripts, introducing DNA into host cells, and performing analyses for assessing expression of the protein of interest and function are known to those skilled in the art. Gene presence, amplification and/or expression may be measured in a sample directly, for example, by conventional Southern blotting, Northern blotting to quantitate the transcription of mRNA, dot blotting (DNA or RNA analysis), or in situ hybridisation, using an appropriately labelled probe which may be based on a sequence provided herein. Those skilled in the art will readily envisage how these methods may be modified, if desired.

Higher eukaryotic cells include insect and vertebrate cells, particularly mammalian cells including human cells or nucleated cells from other multicellular organisms. In recent years propagation of vertebrate cells in culture (tissue culture) has become a routine procedure. Examples of useful mammalian host cell lines are epithelial or fibroblastic cell lines such as Chinese hamster ovary (CHO) cells, NIH 3T3 cells, HeLa cells or 293T cells. The host cells referred to in this disclosure comprise cells in *in vitro* culture as well as cells that are within a host animal.

DNA may be stably incorporated into cells or may be transiently expressed using methods known in the art. Stably transfected mammalian cells may be prepared by transfecting cells with an expression vector having a selectable marker gene, and growing the transfected cells under conditions selective for cells expressing the marker gene. To prepare transient transfectants, mammalian cells are transfected with a reporter gene to monitor transfection efficiency.

To produce such stably or transiently transfected cells, the cells should be transfected with a sufficient amount of the nucleic acid encoding the protein of interest. The precise amounts of DNA encoding the protein of interest may be empirically determined and optimised for a particular cell and assay.

Host cells are transfected or, preferably, transformed with suitable expression or cloning vectors and cultured in the improved cell culture medium. This may be modified as appropriate for inducing promoters, selecting transformants, or amplifying the genes encoding the desired sequences. Heterologous DNA may be introduced into host cells by any method known in the art, such as transfection with a vector encoding a heterologous DNA by the calcium phosphate coprecipitation technique or by electroporation. Numerous methods of transfection are known to the skilled worker in the field. Successful transfection is generally recognised when any indication of the operation of this vector occurs in the host cell. Transformation is achieved using standard techniques appropriate to the particular host cells used.

Incorporation of cloned DNA into a suitable expression vector, transfection of eukaryotic cells with a plasmid vector or a combination of plasmid vectors, each encoding one or more distinct genes or with linear DNA, and selection of transfected cells are well known in the art (see, e.g. Sambrook et al. (1989) Molecular Cloning: A Laboratory Manual, Second Edition, Cold Spring Harbor Laboratory Press).

Transfected or transformed cells are cultured on the improved cell culture medium described here using methods known in the art, preferably under conditions, whereby the protein of interest encoded by the DNA is expressed.

### PROTEIN OF INTEREST

The term "protein of interest" refers to any protein which may be useful for any purpose, particularly research, diagnostic or therapeutic purposes. The protein of interest may comprise a mammalian protein or non-mammalian protein, and may optionally comprise a receptor or a ligand.

Exemplary proteins of interest include, but are not limited to, molecules such as renin; mouse gonadotropin-associated peptide; a microbial protein, such as β-lactamase; epidermal growth factor (EGF); a growth hormone, including human growth hormone and bovine growth hormone; growth hormone releasing factor; osteoinductive factors; immunotoxins; a bone morphogenetic protein (BMP); an interferon such as interferon-α, - β, and -gamma; colony stimulating factors (CSFs), e.g., M-CSF, GM-CSF, and G-CSF; mpl receptor; CTLA-4; protein C; DNase; enkephalinase; RANTES (regulated on activation normally T-cell expressed and secreted); human macrophage inflammatory protein (MIP-1-α); a serum albumin such as human serum albumin; Muellerian-inhibiting substance; relaxin A-chain; relaxin B-chain; prorelaxin; IgE; blood group antigens; flk2/flt3 receptor; obesity (OB) receptor; insulin A-chain; insulin B-chain; interleukins (ILs), e.g., IL-1 to IL-10; transforming growth factor (TGF) such as TGF-α and TGF-β, including TGF-β1, TGF-β2, TG-β3, TGF-β4, or TGF-β5; insulin-like growth factor-I and -II (IGF-I and IGF-II); inhibin; activin; vascular endothelial growth factor (VEGF); IgE; a cytotoxic T-lymphocyte associated antigen (CTLA), such as CTLA-4; lung surfactant.

Other proteins of interest may include a plasminogen activator, such as urokinase or human urine or tissue-type plasminogen activator (t-PA); bombesin; thrombin; hemopoietic growth factor; members of the TNF and TNF receptor (TNFR) family, like tumor necrosis factor-α and -β, CD40 ligand, Apo-2 ligand/TRAIL, DR4, DR5, DcR1, DcR2, DcR3, OPG; receptors for hormones or growth factors; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-β; platelet-derived growth factor (PDGF); T-cell receptors; surface membrane proteins; decay accelerating factor; viral antigen such as, for example, a portion of the AIDS envelope, gp120; transport proteins; homing receptors; addressins; regulatory proteins; integrins such as CD11a, CD11b, CD11c, CD 18, an ICAM, VLA-4 and VCAM; proinsulin; follicle stimulating hormone; calcitonin; luteinizing hormone.

The recombinant cell may be used to express glucagon; parathyroid hormone;. thyroid stimulating hormone; lipoproteins; α-1-antitrypsin; thrombopoietin (TPO); Fas ligand; a tumor associated antigen such as HER2, HER3 or HER4 receptor; antibodies against various protein antigens like CD proteins such as CD3, CD4, CD8, CD19, CD20 and CD34; members of the ErbB receptor family such as the EGF receptor, HER2, HER3 or HER4 receptor; cell adhesion molecules such as LFA-1, Mac1, p150.95, VLA-4, ICAM-1, VCAM and αv/β3 integrin including either α or β subunits thereof (e.g. anti-CD 11a, anti-CD18 or anti-CD 11b antibodies); growth factors such as VEGF; superoxide dismutase; factor; anti-clotting factors such as Protein C; atrial natriuretic factor; des(1-3)-IGF-I (brain IGF-I), insulin-like growth factor binding proteins; CD proteins such as CD3, CD4, CD8, CD 19 and CD20; erythropoietin; clotting factors such as factor VIIIC, factor IX, tissue factor, and von Willebrands fibroblast growth factor such as aFGF and bFGF; or an Apo-2L receptor such as Apo-2 (DR5), DR4, DcR1, DcR2, DcR3.

In a particularly advantageous embodiment, the protein of interest comprises a therapeutic polypeptide or a therapeutic antibody. Preferably, the therapeutic antibody comprises a monoclonal antibody. Preferred therapeutic monoclonal antibodies are those that are targeted to, for example, CD20, her2?neu, CD52, VEGF, EGF receptor, CD33, CD20, CEA, EpCAM, GD2, Le(Y), TNF.

Variants and/or fragments of any of the above are also included.

### THYMIDINE FAMILY MEMBER

The improved cell culture medium comprises an elevated concentration of a thymidine family member. As the term is used in this document, "thymidine family member" refers to a compound having the following structure: 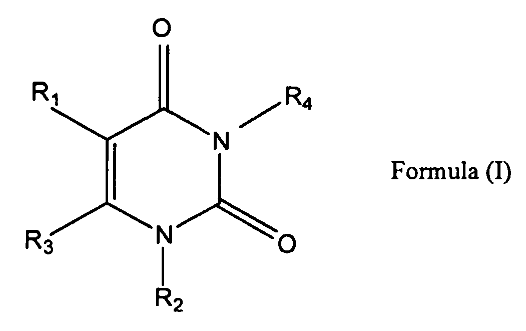 where R1, R2, R3 and R4 independently comprise a hydrocarbyl group, as defined below.

Preferably, R1 comprises an alkyl group, more preferably a lower alkyl group. More preferably, R1 comprises an butyl, propyl, ethyl or methyl group. Most preferably, R1 comprises a methyl group.

Preferably, R2 comprises a sugar or a derivative thereof. More preferably, R2 comprises a monosaccharide, preferably an aldose or a pentose. Most preferably, R2 comprises ribose or deoxyribose.

Preferably, R3 and R4 comprise hyrdogen.

The term "thymidine family member" therefore includes thymidine, uridine, a thymidine analogue and a uridine analogue.

In some embodiments, the thymidine family member comprises a compound of formula (I), in which R1 is methyl and R2 is deoxyribose. Thus, in such embodiments, the thymidine family member comprises thymidine or a thymidine analogue (as described in further detail below).

In other embodiments, the thymidine family member comprises a compound of formula (I), in which R1 is hydrogen and R2 is ribose. Thus, in such embodiments, the thymidine family member comprises uridine or a uridine analogue (as described in further detail below).

It will furthermore be clear from this document that the composition of the improved cell culture medium is not limited to the presence of a single thymidine family member, and that more than one thymidine family member may be present. Thus, it is specifically contemplated that the improved cell culture medium contain two or more thymidine family members, preferably chosen from a combination of:
(i) thymidine and a thymidine analogue;
(ii) uridine and a uridine analogue;
(iii) a thymidine analogue and a uridine analogue; and
(iv) thymidine and uridine.

### THYMIDINE

The term "thymidine" refers to thymidine itself.

In some embodiments, the thymidine family member comprises thymidine, i.e., 1-[4-hydroxy-5-(hydroxymethyl)oxolan-2-yl]-5-methyl-pyrimidine-2,4-dione having CIS number 1134.

The thymidine may comprise any isomer thereof, e.g., thymidine (CIS: 5789) 1-[(2R,4S,SR)-4-hydroxy-5-(hydroxymethyl)oxolan-2-yl]-5-methyl-pyrimidine-2,4-dione or L-thymidine or thymidine (CIS: 159269) 1-[(2S,4R,5S)-4-hydroxy-5-(hydroxymethyl)oxolan-2-yl]-5-methyl-pyrimidine-2,4-dione.

### THYMIDINE ANALOGUE

In some embodiments, the thymidine family member may comprise a thymidine analogue, by which term is meant a molecule chemically or functionally related to thymidine comprising one or more substituents. Preferably, the thymidine analogue comprises one or more properties of thymidine, preferably that it is capable of growth enhancement or cell viability improvement activity.

Thymidine analogues which may be suitable for use in the improved cell culture medium include any of the following (CIS numbers in brackets following):
Thymidylic acid (1139); thymidylic acid (9700); dTTP (64968); Telbivudine (159269); pdTp (121976); Thymidine-alpha-t (119209); deoxy-TDP (1124); deoxy-TDP (164628); Thymidine-methyl-t3 (162681); 14C-Thymidine (134556); Thymidine-6-t (134667); dTTP (1151); Thymidine der. (475072); Thymidine & AZT (451805); Thymidine glycol (3035041); Monotritylthymidine (98082); Dideoxythymidine (65119); dTDP-L-rhamnose (121966); 3-(3-Oxopropenyl)thymidine (128983); 5'-Dimethoxytrityl-thymidine (627554); Thymidine, 3',5'-diacetate (98007); Thymidine-5'-aldehyde (3081398); 5'-Tosylthymidine (98028); alpha-Thymidine (5270603); Thymidine C5-hydrate (115160); Acyclo-thymidine (451953); Acyclo-thymidine (451449); d(Tpst) (3082437); Thymidine bromohydrin (170741); Thymidine deriv. (465324); Thymidine deriv. (465319); 3-(2-Cyanoethyl)thymidine (126306); 5'-O-(Myristoyl)thymidine (474370); 4-Thiothymidine (278191); Thymidine 5'-O-pivaloate (100521); Cyclic tmp (461278); Thioacyclo-thymidine (451954); Thioacyclo-thymidine (451952); Carbathymidine (130766); 5'-O-Myristoyl thymidine der. (474375); 5'-O-Myristoyl thymidine der. (474371); dTpdT (3035025); Thymidine-2-14C (166753); 5'-O-Myristoyl thymidine der. (474377); 5'-O-Myristoyl thymidine der. (474376); 5'-O-Myristoyl thymidine der. (474373); 5'-O-Myristoyl thymidine der. (474372); 1-Dpft (72247); 4'-Azidothymidine (72296); 2-Thiothymidine (3005944); Thymidine-alpha-14C (166772); 5'-O-Myristoyl thymidine der. (474374); zidovudine (35370); N3-Ethylthymidine (159912); THYMIDINE-3'-PHOSPHATE (444920); Thymidine 3'-benzoate (87056); Thymidine,14C labeled (169201); Thymidine, 3-propyl- (155377); Thymidine, 3-chloro-(152387); Methylthymidine (65062); 3'-Thiothymidine (124411); 4'-Thiothymidine (72371); 4'-Cyanothymidine (452277); 3'-O-Ph-SC=O-2'-T (451885); Thymidine, P-methylthymidylyl-(3'-5')- (189096); 5'-Deoxythymidine (65120); Dttp alphas (161258); Ddt(3'nhme) (3035713); OMET (72250); 4',5-Acyclo-thymidine (451951); dTpdU (165861); 4-O-Ethylthymidine (107988); Dtp(Et)dT (3081472); alpha-Fluorothymidine (127931); PNPpT (164953); O-3',5'-Bis(trifluoroacetyl)thymidine (581023); 3'-Acetylamino-3'-dT (452680); MMUdR (119051); 3'-Isocyano-dT (72272); 2'-Dcpt (165270); 3'-OEt-ddT (451832); thymidine-5'-monophosphate (444919); O6,5'-Cyclothymidine (557302); 3,5-thymidine diphosphate (1618); stavudine (18283); Oxetane deriv. (453632); 5,6-Dihydrothymidine (165328); Dttp-gly (129192); Adrt-TP (122637); Pptppp (194545); 3'-OCOONa-ddT (451833); Thymidine, 4'-C-ethynyl- (451503); 4MUpT (196156); beta-Ghmu (124162); Thymidine, 3'-azido-3'-deoxy- (5275789); 5'-O-Dimethyltritylthymidine (162419); 5'-Iat (127792); Thymidine-5'-carboxylic acid (330079); Thymidine glycol monophosphate (165415); Thymidine-5'-carboxylic acid (100533); BH3dTTP (3005830); alpha-Hydroperoxythymidine (148264); (+)-6'-Methyl-carba-thymidine (370698); 3',5'-Dideoxythymidine (168859); 4'-(Trifluoromethyl)thymidine (470800); (-)-3'-epi-carba-thymidine (370697); 5'-Amino-5'-deoxythymidine (297924); 3'-Aminothymidine (108074); 5'-Bromoacetamido-5'-deoxythymidine (100092); Cyanothymidine (72265); 5'-Deoxy-5'-(1-thyminyl)thymidine (557306); 5'-diBu-PO4T (452555); beta.-L-ddT (451359); N(3)-Benzylthymidine (3082190); Thymidine 5'-phospho-alpha-naphthylamidate (191228); N(3)-Allylthymidine (128451); O-Methylthymidine (73316); 5'-Ph AZT deriv. (452994); 3'-Mesyl-2'-dT (451841); Ddthds (3000253); Thymidine, 2',3'-didehydro- (152369); 3'-Cl-ddT (72249); Thymidine, 5'-ammonium deriv. (462769); Thymidine, 5'-ammonium deriv. (462768); Thymidine, 5'-ammonium deriv. (462767); Thymidine, 5'-ammonium deriv. (462766); Thymidine, 5'-ammonium deriv. (462765); Thymidine, 5'-ammonium deriv. (462764); Thymidine, 5'-ammonium deriv. (462763); Thymidine, 5'-ammonium deriv. (462762); Thymidine, 5'-ammonium deriv. (462761); Thymidine, 5'-ammonium deriv. (462760); 5'-diPr-PO4T (452554); Methm-deoxyuridine (135518); ddTTP (65051); DTDS (130348); 3'-I-ddT (451455); Tmpnpp (3081855); Trifluridine (253083); F2-Tdr (130510); 3'-Deoxy-3'-iodothymidine (72197); 2-OHMe-1,3-OxathiolanT (454509); NSC308308 (328672); α-L-4'-AZT (3001563); EINECS 248-679-4 (119623); 3'-O-ddT (72263); zidovudine (5726); 4'-AzdT (453830); 3'-Formamido dT (452132); 3'-Br-ddT (451830); 5'-N3-5-Me-ddU (451456); TTCEP (3082139); Thymidylyl-(3'-5')-uridine (192922); North-Methanocarbathymine (445212); 3'-O-Acetyl-4-thiothymidine (3037732); 5'-Fluorodeoxythymidine (285661); 3-Dhp-dthd (130324); 5HOMedU (91541); AIDS060432 (469805); Alovudine (33039); CHEMBANK907543 (5463); 5-Hydroxy-d4T (196956); Thymidine, alpha-(methylsulfonyl)- (153548); AIDS049978 (466467); CHEMBANK907540 (5464); 3'-Acetylthio-ddT (451882); 3'-SCN-ddT (451837); 3'-SEt-ddT (451835); 3-OMe-CycloSal-d4TMP (451504); NISTR144715 (524948); 5'-IsocyanoT deriv. (452960); 3'-CH3S-dT (452397); ddC & T (451755); 5-Icdt (122648); AIDS001007 (451898); NSC641699 (369676); NSC365429 (339327); 4-Thiothymidine (3034569); NSC98676 (263957); 3'-(5-MeTriazol)dT (452537); [(S)-Methanocarba-T] (447628); NSC658787 (377346); Thymidine, 5,6-dihydro-, (5R)- (157173); ddTHd (72230); NISTR144720 (524949); NISTR144700 (524947); NISTR144691 (524945); AIDS051834 (467414); Anhydro-trityl-T (453037); 3-pyrazoldT (452138); THYMIDINE-5'-(DITHIO)PHOSPHATE (5289467); AIDS007344 (456203); 2',3'-Dideoxyuridine deriv. (453112); 2',3'-Dideoxyuridine deriv. (453111); 3'-(DiMeTriazol)dT (452535); 3'-ImidazoldT (452139); Ap(5)dT (447779); d(Apt) (3080727); Mmacdurd (196676); Thymidine 5'-monophosphate sodium salt (153672); 3'-O-tert-butyldimethylsilylthymidine (552460); Trifluridine (6256); dd-CF3-ddT (453199); Ph-Se-CF3-ddT (453197); NSC641698 (438518); NSC646438 (371875); WHI-01 (367709); NSC225110 (312899); 3-Adtpp (146124); F2dT (72257); AIDS169811 (506492); AIDS070488 (470798); 3'-Cyanamino-3'-dT (452695); 3'-isothiocyano dT (452421); Methylene-Az-ddTDP (451978); 3'-Hydroxymethylthymidine (3000763); N3ddThdS (3000254); 3'-Acetamido-3'-deoxythymidine (122641); 3'-O-MeSCS-T (454195); ECP-AZT (452551); NSC374909 (341732); 3'-F-4-Thio-ddT (3000239); THMPT (195200); 5'-O-(tertbutyldimethylsilyl)-thymidine (557070); 3'-5-MeTriazol-dTTP (452540); 3'-isoselenocyano ddT (452422); 3'-CH2COOH-ddT (451967); NSC312330 (329450); dTpdC (168992); BH3-d4TTP (456196); Rp-α-Borano-AZT-TP (455767); 5',4'-iprdiOT (453636); 2',3'-Dideoxyuridine deriv. (453114); 3'-Morpholino-dT (452740); 3'-Methylsulfonyl-dT (452399); NSC268672 (320565); AIDS000557 (3000240); 5'MeOValPO3(Bu)T (452580); 3'-DiMeTriazol-dTTP (452539); 3'-S-ddT dimer (451883); AIDS000955 (451858); THYMIDINE-5'-DIPHOSPHO-BETA-D-XYLOSE (447462); d(Apgpcpt) (3081621); TPCET (126666); Oxetane deriv. (453633); 5'-O-Acetyl AZT (452423); 3'-Tolylthio-dT (451884); 3'-SCH2CH2OH-ddT (451836); ddC-AZT dimer (451582); NSC365495 (435211); NSC365494 (435210); Thymidine, 5'-azido-5'-deoxy-, 3'-acetate (579901); Thymidine, 5'-amino-5'-deoxy-, 3'-acetate (539396); AIDS 155063 (497663); 2',3'-Dideoxyuridine deriv. (453115); Acetyl-FLT (453016); 5'-O-Decanoyl AZT (452427); 5'-O-Octanoyl AZT (452426); 5'-O-Hexanoyl AZT (452425); 5'-O-Butyryl AZT (452424); 3'-(1,2,3-Triazol)dT (452142); 2'-(1,2,4-triazol)dT (452141); 3'-(1,2,4-triazol)dT (452140); AIDS001289 (452133); NSC238143 (315104); 3'-Fluorothymidine (284240); 5-Addot (65311); 3'-Benzylamino-dT (452741); 3'-Valerylamino-3'-dT (452685); 3'-Butyrylamino-3'-dT (452683); 3'-Propionylamino-3'-dT (452682); 3'-NH23-5-CF3-ddU (451856); Zidovudine sodium salt (3035471); Ddethds (3000238); Dtpdfu (166788); 5'-Fluorodeoxythymidine (97675); β.-L-D4T (470154); ddT-HP (454590); 3-2,3EpoxyPrAZT (454168); T-5'PNPOP (454113); a.-4'AzMeD4T (453641); AZT deriv. (453201); 5'-IsocyanoAZT deriv. (452962); 5'-Phosphonomethylene-3'-AzdT (452206); 3'-Me-D4T (452030); 3'-Azido-3'-deoxythymidine 5'hexadecanoate (153849); 3-Aiddt (122650); AIDS083699 (474759); 3'-Trifluoroacetylamino-3'-dT (452681); 3'-FD4T (451566); NSC654956 (375342); Thymidine, 5-deoxy-5-(4-methoxytritylaminomethyl)- (623452); d4T & Costatolide (474561); 3(3Me-2Butenyl)AZT (454171); Mesyl-FLT (453014); 5'-benzoyl-D4T (452583); 5'-capryl-D4T (452581); 3'-Methylsulfinyl-dT (452398); 5'(H2PO3CH2)AZT (452127); 5'-acetyl-D4T (451499); NSC637643 (438432); 3-Mdttp (131885); d4T & Calanolide A (474549); 2'-DeoxyC & AZT (451809); ddT-MeP (454602); AIDS002269 (452700); Thymine acyclonucleoside (452443); AIDS001175 (452040); dTDP-alpha-D-glucose (443210); FdTTP (162005); Thymidine, 2',3'-didehydro-alpha,alpha,alphatrifluoro- (152380); TMPTE (125186); BDERT (124147); AZT-triphospate (72187); AIDS123013 (493548); Phosphonate deriv. of AZT (489870); Phosphonate deriv. of AZT (489869); Phosphonate deriv. of AZT (489867); d4EMUrd (473844); AZT der. (471099); AIDS070489 (470799); AZT & SAM (469688); CQ & AZT & HU (457861); AZT & FUdR (457708); AIDS008748 (456605); AZT deriv. (453173); 3'-Isobutyrylamino-3'-dT (452684); Phosphazid (452228); N(CH2COAZT)morpholine (452193); NSC647643 (438622); AIDS002256 (3000306); 3'-(3-Ethylthioureido)-3'-dT (3000305); Hmdutp (159726); Dddttp (65355); AIDS084971 (474830); d4T & Dihydrocostatolide (474572); AIDS071183 (471294); Rifamycin analog (468376); ZDV & Didanosine (451772); ddC & AZT (451759); 2',3'-DEHYDRO-2',3'-DEOXY-THYMIDINE 5'-TRIPHOSPHATE (5288019); AZT-bis(3NO2Ph)PO4 (455298); AZT-bis(2NO2Ph)PO4 (455297); FLT-HP (454600); AIDS001569 (452272); DiNa-5'-O-Phosphonomethyl-dT (452266); AIDS001104 (451980); NSC659923 (378122); NSC659922 (378121); NSC659921 (378120); NSC657733 (376690); NSC657732 (376689); NSC619502 (359060); 5'-(4-Cyano-1H-1,2,3-triazol-1-yl)-5'-deoxythymidine (171232); 3-Gamt (159513); AZTDP (129170); 3-Bdtfu (125883); Phosphatidyl AZT (122103); AZT glucuronide (105108); L-FTTP (497307); AZT & AZC (487172); AIDS093771 (479211); AIDS093769 (479209); AZT der. (471098); AZT der. (471097); AZT der. (471096); AZT der. (471095); AZT der. (471094); AZT der. (471092); AZT der. (471091); AZT der. (471090); AZT der. (471089); AZT der. (471088); AZT der. (471087); AZT der. (471086); AZT der. (471085); AZT der. (471084); AZT der. (471083); AZT der. (471082); AZT der. (471081); AZT der. (471080); AZT der. (471079); AZT der. (471078); L-3'-FD4T (464993); D4T-DP (461279); dipyridamole (3108); Uridine & AZT (451762); 2*-DEOXY-THYMIDINE-5*-ALPHA BORANO DIPHOSPHATE (ISOMER RP) (5289440); FLT-MeP (454599); d4T-HP (454594); OligopyranSPO3H2-T (454447); T-5'PNPNP (454116); 2',3'-Dideoxyuridine deriv. (453109); PrNHPO3(Et)AZT (452712); AIDS002270 (452701); 3'-(4,5-bisCarboxytriazole)dT (452380); AZT-MeP (452209); AIDS001176 (452041); NSC659017 (377522); NSC659015 (377520); NSC637642 (367710); 3'-F-Dtfp (3081896); AIDS224684 (3013716); AIDS224682 (3013714); 3'-(3-Allylthioureido)-3'-dT (3000311); AIDS002259 (3000309); 5'-(4-Carboxy-1H-1,2,3-triazol-1-yl)-5'-deoxythymidine (171231); d(Cgt) (125783); Azt-cds (72184); AIDS 181946 (510144); AIDS 160934 (500169); AZT 5Br6iPrO deriv. (500167); AIDS093772 (479212); AIDS093703 (479156); Amino acid carbamate deriv (478209); Amino acid carbamate deriv (478208); Amino acid carbamate deriv (478206); Amino acid carbamate deriv (478205); Amino acid carbamate deriv (478204); AIDS070845 (471077); AIDS070329 (470649); D4CT 5',5-OPO3H2 (454935); D4AT 5',5-OPO3H2 (454934); 2',3'-Dideoxyuridine deriv. (453110); AIDS002783 (453038); Ether lipid (452650); 5'-pivaloyl-D4T (452584); AIDS001745 (452387); 3'-(4,5-bisMeOH-triazole)dT (452376); AIDS001571 (452275); AIDS001570 (452274); Bu2MeSiCNddT (452056); bis-AZT Phosphate (451440); 3'-F2-dTTP (451385); AIDS000047 (451383); NSC637646 (367713); NSC619504 (359063); AIDS224683 (3013715); AIDS002263 (3000313); AIDS002262 (3000312); AIDS002257 (3000307); d(A-T-G-T) (196171); N(3)-Meazttp (164449); 3'-Ctnu (125188); 5'-O-(4,4'-Dimethoxytrityl)-3'-O-(4-oxopentanoyl)-2'-desoxy-thymidine (627549); Thymidine, 5'-deoxy-5'-(phosphonoamino)-, bis(2,2,2-trichloroethyl) ester (581038); Thymidine, 3'-O-(trimethylsilyl)-, 5'-[bis(trimethylsilyl) phosphate] (557067); AIDS 191298 (515190); AIDS 160592 (500092); AIDS093770 (479210); Amino acid carbamate deriv (478207); AIDS085805 (475352); Adefovir & d4T (473388); AIDS080588 (473284); AIDS080582 (473278); AZT der. (471093); AIDS058264 (468373); AIDS058263 (468372); AIDS058259 (468368); AIDS051889 (467455); AIDS051888 (467454); AIDS051887 (467453); 2'-DeoxyG & AZT (451808); 2'-ddA & AZT (451807); Cytidine & AZT (451804); Sulfadiazine & AZT (451803); Pyrimethamine & AZT (451802); AZT & Prasterone (451771); AZT & Ganciclovir (451763); Foscarnet & AZT (451561); b,g-CF2-AzTTP (453454); 3'BzNH2-5'(4MeOTrityl)dT (452739); 3'Morph-5'(4MeOTrityl)dT (452738); MeOCOPentNHPO3(Et)AZT (452711); AIDS002290 (452710); MeOCOPrNHPO3(Et)AZT (452709); MeOAlaNHPO3(Et)AZT (452708); Ether lipid (452652); Ether lipid (452648); Glc-hexyl-CO3 AZT (452553); 3'-(4,5-EtOCO-triazole)dT (452351); AIDS001729 (452350); AZT-PO3(CH3)-AZT (452257); ddTTP & PFA (451811); AZTTP & Foscarnet (451770); AZT-DHP QS (451381); NSC646436 (371874); NSC619503 (359061); HOAc-AZT (3081313); PPi+AZTTP (3013717); 3'(3Adamanthylthioureido)-3'dT (3000310); AIDS002258 (3000308); 5-Dctnps (196161); Vitacic (196118); 5'-(4,5-Bis(methoxycarbonyl)-1H-1,2,3-triazol-1-yl)-5'-deoxythymidine (171230); Bis-5'-D4T phosphate (164466); d-Tmpp (164414); Abbg-DM-DTTP (146130); EINECS 261-992-0 (108861); AIDS320301 (5279488); AIDS320300 (5279487); AIDS293204 (5279007); AZTTP+ATP (5274824); AIDS 160758 (500109); AIDS 123150 (493614); AZT & OMG-PFA (489586); AZT & ODG-PFA (489585); Sulfadiazine & D4T (479898); Pyrimethamine & D4T (479890); AIDS088188 (477072); PMPA & d4T (473396); AIDS058262 (468371); AIDS058261 (468370); 2'-Deoxyuridine & AZT (451806); AZT & N-Butyl-DNJ (451768); Adefovir & AZT (451764); Avarol & AZT (451761); Inosine-Pranobex (451760); ACV & AZT (451758); AZT & Ansamycin (451757); Ribavirin & AZT (451752); Potenlini & AZT (451750); Persantin & AZT (451734); AZT & 6-Thiodeazaguanine (3000244); AIDS007658 (456260); AIDS005919 (455320); AZT-5'-steroid ester (454275); AZT-5'-steroid ester (454274); AZT-5'-steroid ester (454273); 2',5'diSilySpiroT (453846); 2',5'diSilySpiroT (453845); AcNHGlc-hexyl-CO3 AZT (452552); 5'-O-Triphosphomethyl-3-FdT (452276); Methylene-AzddTTP (451979); AZTdpdg (451738); NSC643408 (370347); NSC640323 (368918); 3-Dttctp (3083403); NH2d(Tcmt) (3083179); Dmt-tdmtt (3082939); AZTTP-DSG (3081908); CHOAD-AZT (3081314); 3dTDP-Dmg (3036093); PPi+EFV+d4TTP (3013719); PPi+EFV+AZTTP (3013718); 4'-Ethynyl D4T + dThd (3013328); 6-TG + SQV + AZT (3013142); AIDS218075 (3011706); AIDS218035 (3011666); AIDS218034 (3011665); AIDS218033 (3011664); AIDS218032 (3011663); AIDS218031 (3011662); AIDS218030 (3011661); AIDS192875 (3007080); CADA and Stavudine (3006561); CADA and AZT (3006560); AIDS080887 (3001960); UC-10 & d4T (3001947); AIDS025484 (3000791); d(T-A-A-T) (196167); DHTFT (191569); dTDP-L-mycarose (191004); d-Ftmpp (164421); Combivir (160352); Trizivir (160351); 3dTDP-Pyrdg (160039); 83199-32-0 (157932); Cemabga-dttp (125779); Cyclo-d4G+AZT (5279630); Cyclo-d4G+d4T (5279627); AIDS320319 (5279506); AIDS320318 (5279505); AIDS320317 (5279504); AIDS320316 (5279503); AIDS320315 (5279502); AIDS320314 (5279501); AIDS320313 (5279500); AIDS320312 (5279499); AIDS320311 (5279498); AIDS320310 (5279497); AIDS320309 (5279496); AIDS320308 (5279495); AIDS320307 (5279494); AIDS320306 (5279493); AIDS320305 (5279492); AIDS320304 (5279491); AIDS320303 (5279490); AIDS320302 (5279489); AIDS320299 (5279486); AIDS320298 (5279485); AIDS320297 (5279484); Mesuol+AZT (5277588); PPi+AZTTP+ddTTP (5277573); AIDS257733 (5277507); AIDS241532 (5276904); AIDS228342 (5275767); AZT+4-Arylmethylpyridinone (5274825); TAK-652+AZT (5274627); AIDS226188 (5274152); AIDS226187 (5274151); AIDS226186 (5274150); AZTTP + PPi + Nevirapine (5274077); AIDS 160659 (500099); AIDS 155066 (497666); AIDS 155065 (497665); AIDS 155064 (497664); AIDS 153202 (496802); Efavirenz & AZTTP (492399); AIDS105894 (484335); Mycophenolic acid & D4T (477591); (-)-Carbovir & d4T (477563); Capravirine & Zidovudine (477515); D4T & L-D4FC (475833); AIDS081442 (473852); AIDS081441 (473851); AIDS081440 (473850); AIDS081439 (473849); AIDS081383 (473811); AIDS081382 (473809); AIDS080907 (473445); AIDS080906 (473444); 3TC & TSAO-m^3T (473441); AIDS071447 (471522); AIDS071446 (471521); AIDS071445 (471520); AIDS071444 (471519); AIDS071439 (471514); AIDS071415 (471490); AIDS071414 (471489); AIDS071413 (471488); AIDS058260 (468369); AZT & Heme (467807); AZT & AM-18-OEt (467806); D4T & SRR-SB3 (466498); AZT-5'-steroid ester (464753); AIDS030866 (461998); Persantin & ddC (451810); AZT & Hypericin (451789); Ribavirin & D4T (451753); Bromoouridine (236184); Broxuridine (6035); stavudine (5280).

### URIDINE

The term "uridine" refers to uridine itself.

In some embodiments, the thymidine family member comprises uridine, i.e., 1-[3,4-dihydroxy-5-(hydroxymethyl)oxolan-2-yl]pyrimidine-2,4-dione having CIS number 1177.

The uridine may comprise any isomer thereof, e.g., CIS 6029: 1-[(2R,3R,4R,5R)-3,4-dihydroxy-5-(hydroxymethyl)oxolan-2-yl]pyrimidine-2,4-dione or CIS 466466: 1-[(25,3S,4S,5S)-3,4-dihydroxy-5-(hydroxymethyl)oxolan-2-yl]pyrimidine-2,4-dione.

### URIDINE ANALOGUE

In some embodiments, the thymidine family member may comprise a thymidine analogue, by which term is meant a molecule chemically or functionally related to thymidine comprising one or more substituents. Preferably, the thymidine analogue comprises one or more properties of thymidine, preferably that it is capable of growth enhancement or cell viability improvement activity.

Uridine analogues which may be suitable for use in the improved cell culture medium include any of the following (CIS numbers in brackets following):

2,3(1H,3H)-Pyrimidinedione, 1-beta-L-arabinofuranosyl- (170274); uridine (6029); 1-[(3R,4R,SR)-3,4-dihydroxy-5-(hydroxymethyl)oxolan-2-yl]pyrimidine-2,4-dione (643973); Spongouridine (18323); ZINC01446448 (1521749); 4-hydroxy-1-pentofuranosylpyrimidin-2(1H)-one (638039); uridine (466466); Ara-Furan-U (453551); Uridine-5-t (165148); Uridine-5,6-t2 (151772); Uridine-6-t (167877); 3'-Deoxyuridine (145984); 3'-O-Methyluridine (560146); 1-[3-hydroxy-5-(hydroxymethyl)oxolan-2-yl]pyrimidine-2,4-dione (4085802); deoxyuridine (453556); ZINC01075672 (1263358); ZINC00056350 (687971); deoxyuridine (13712); 2'-O-Methyluridine (102212); deoxyuridine (640); 2'-O-Methyluridine (560213); 1-[(4S,5R)-4-hydroxy-5-(hydroxymethyl)oxolan-2-yl]pyrimidine-2,4-dione (644272); NSC666700 (380916); 1-(B-D-glucofuranosyl)uracil (356730); AIDS144387 (495716); 3'-Deoxy-3'-fluorouridine (451602); NSC148459 (288045); NSC150822 (289160); 2'-Fluoro-2'-deoxyuridine (150851); 3-I-Ara-U (167800); 4'-Azidouridine (453828); ZINC00120602 (719815); ZINC01431207 (1510822); ZINC00120599 (452809); FEAC (3007908); NSC291045 (324745); AIDS 189190 (513589); AIDS047737 (5270567); β.-L-ddU (451413); ddUrd (65161); 2',3'-Dideoxyuridine (355836); MLS000084853 (3236176); 2'-Fluor-uridine (352500); CHEMBANK852837 (1343); Uridine analog (500903); 3'-Hydroxymethyl Lyxo U (452417); 3'-C-Methyluridine (5270589); 3-Methyluridine (316991); AzdU deriv. (452990); 3-Methyluridine (99592); 6-amino-3-[3,4-dihydroxy-5-(hydroxymethyl)oxolan-2-yl]-1H-pyrimidine-2,4-dione (5256480); 1-(3,4-dihydroxy-5-methyl-oxolan-2-yl)pyrimidine-2,4-dione (3711764); 3'-Hydroxymethyl Ara U (452408); 2'-Iodo-2'-deoxyuridine (188319); MOLI000884 (450447); NSC525628 (352281); NSC529445 (352980); ZINC01078616 (1265896); AIDS184958 (510970); 2',3'-Anhydrolyxo-U (451886); 5-fluoro-5'-deoxyuridine (1817); MOLI001294 (450760); 1-[(2R,3R,5R)-3,4-dihydroxy-5-(hydroxymethyl)oxolan-2-yl]-5-hydroxy-pyrimidine-2,4-dione (2729604); 1-[4-fluoro-5-(hydroxymethyl)oxolan-2-yl]pyrimidine-2,4-dione (3859105); ZINC01081178 (1268076); 2'F-dd-araU (452032); MOLI000592 (450215); L-5-Fluorouridine (5270526); 5-Fluorouridine (9427); 3'-O-formyl2'-dU (454121); AIDS080589 (473285); 3'-Me-araU (451893); 5-Icddu (122649); AIDS047705 (466402); AIDS002637 (452961); 5-Hydroxyuridine (94196); 5-Fluorouracil arabinoside (101317); 1-[(2R,5R)-4-fluoro-5-(hydroxymethyl)oxolan-2-yl]pyrimidine-2,4-dione (2783888); NSC82265 (256040); 5-Fluorouridine (1821); 5'-Amino-2'-deoxyuridine (451827); AIDS096370 (481086); AIDS080583 (473279); NSC639758 (368644); NSC152516 (289972); NSC175992 (300719); ZINC01444205 (1520351); Fddurd (162450); Uridine 3'-spiroxirane (472365); oh-5-dU (96622); 3-Hydroxyethyldeoxyuridine (130680); NSC129945 (279434); 1-[5-(dihydroxyphosphinothioyloxymethyl)-3,4-dihydroxy-oxolan-2-yl]pyrimidine-2,4-dione (3364664); URIDINE-2',3'-VANADATE (5289542); 5-Hydroxyuridine (1828); NSC260813 (319152); AIDS191148 (515083); 3-Dhp-durd (124412); NSC639425 (368423); ZINC01075517 (1263225); 3'-Uridylic acid (101543); ZINC00150399 (734628); ZINC01239126 (1384327); Uridine Vanadate (446487); [(2R,3R,4S,5S)-5-(2,4-dioxopyrimidin-1-yl)-3,4-dihydroxy-oxolan-2-yl]methoxyphosphonic acid (2069168); 1-[3,4-dihydroxy-5-[(hydroxy-oxido-phosphoryl)oxymethyl]oxolan-2-yl]pyrimidine-2,4-dione (3780171); 3'-isocyano-ddU (72273); 1-[3,4-dihydroxy-5-(phosphonatooxymethyl)oxolan-2-yl]pyrimidine-2,4-dione (4014956); AMddU (122107); [4-acetyloxy-5-(2,4-dioxopyrimidin-1-yl)-2-(hydroxymethyl)oxolan-3-yl] acetate (4102969); 1-[3,4-dihydroxy-5-(hydroxymethyl)oxolan-2-yl]pyrimidine-2,4-dione; oxovanadium; dihydrate (3393377); uridylate (1172); 3'-N3-araU (451890); URACIL ARABINOSE-3'-PHOSPHATE (5289522); ZINC02123545 (1778309); ZINC02606132 (2069167); (211At)Audr (124994); NSC401847 (344757); [(2R,3R,4S,5R)-5-(2,4-dioxopyrimidin-1-yl)-3,4-dihydroxy-oxolan-2-yl]methoxyphosphonic acid (18817); NSC268654 (320548); Uridine 3-monophosphate (5643); Uridylic acid (6030); N-[5-(2,4-dioxopyrimidin-1-yl)-4-hydroxy-2-(hydroxymethyl)oxolan-3-yl]ethanamide (4471189); 3-Br-Ara-U (165421); Uridine 2'-phosphate (101609); NSC268662 (320555); 3'-Hydroxymethyl ddU (452407); ZINC02606131 (2069166); Uridine 2-monophosphate (5642); 5'-Iod-uridine (352979); HgdUrd (511713); EUrd (176869); I-araU (96621); A3104/0131463 (5175675); [5-(2,4-dioxopyrimidin-1-yl)-3,4-dihydroxy-oxolan-2-yl]methoxy-(oxido-phosphonatooxy-phosphoryl)oxy-phosphinic acid (5058929); 5-Aminouridine (251784); ZINC01075220 (1262955); ZINC00041578 (677806); 5-Iodouridine (256025); uridine triphosphate (5644); [[5-(2,4-dioxopyrimidin-1-yl)-3,4-dihydroxy-oxolan-2-yl]methoxy-hydroxy-phosphoryl]oxyphosphonic acid (3084486); 2'-Bromo-2'-deoxyuridine (188257); 2',3'-diF-arabino-ddU (452215); 5'-Iodo-2'deoxyuridine (188325); [[5-(2,4-dioxopyrimidin-1-yl)-3,4-dihydroxy-oxolan-2-yl]methoxy-hydroxy-phosphoryl]oxyphosphonic acid (5009466); Ara-utp (151607); NSC263612 (319415); UDP (6031); NSC646939 (372074); 3-Cl-Ara-U (101508); Uteplex (6133); 2'-5'dideoxyuridine (1465); N(3)-Allyluridine (128450); NSC697730 (394348); ZINC00106013 (712748); U5P (446164); [5-(2,4-dioxopyrimidin-1-yl)-3,4-dihydroxy-oxolan-2-yl]methoxy-phosphonatooxy-phosphinic acid (4994354); 1-(2,5-dideoxy-beta-L-erythro-pentofuranosyl)pyrimidine-2,4(1H,3H)-dione (643514); AIDS226344 (5274316); AIDS218471 (5272753); 3'-Chloro-3'-deoxyuridine (3080715); [5-(2,4-dioxopyrimidin-1-yl)-3,4-dihydroxy-oxolan-2-yl]methyl 2,2-dimethylpropanoate (3570151); 1-[(2R,3R,5R)-3,4-dihydroxy-5-(hydroxymethyl)oxolan-2-yl]-5-iodopyrimidine-2,4-dione (2729605); uridine-diphosphate (1158); NSC687952 (390512); Bracurd (3082267); NSC529438 (352977); NSC264399 (429777); 5-Iodouridine (1268108); 2'-5'dideoxyuridine (444320); AIDS226181 (5274145); uridine-triphosphate (1181); 3'-I-ddU (451454); 3'-Deoxy-5-fluorouridine (5273019); Uridine, 2-thio-(3036443); AIDS047643 (3001487); 2',3'-F2-ddU (452035); 2',3',5'-Tri-O-methyluridine (142387); AIDS185207 (511158); ZINC00001457 (667486); NSC91437 (260345); UMP sodium salt (169020); 5-fluoro-1-[(4S,5R)-4-hydroxy-5-(hydroxymethyl)oxolan-2-yl]pyrimidine-2,4-dione (643968); dUMP (65063); AIDS114155 (490449); NSC648550 (372860); 5'-Azido-2'-deoxyuridine (451825); dUMP (688); 1-Deoxy-1-(3,4-dihydro-2,4-dioxo-1(2H)-pyrimidinyl)-beta-D-ribofuranurononitrile (150628); Uracil deriv. (452050); AZddU (threo) (452029); 2'-Azido-2'-deoxyuridine (386489); AIDS226185 (5274149); AIDS 181188 (509513); ZINC00489135 (907229); 3NH-D-arab-ddU deriv. (452877); 2'-Deoxyuridine 5'-monophosphate (446900); NSC677989 (386248); [5-(2,4-dioxopyrimidin-1-yl)-3-hydroxy-4-methoxy-oxolan-2-yl]methoxyphosphonic acid (3274683); ZINC00388678 (853012); 3NH-D-ribo-ddU deriv. (452890); AIDS169808 (506489); floxuridine (5790); 5-Fluorouracil 3-riboside (295499); floxuridine (3363); 2'-Azido-2'-deoxyuridine (168629); MOLI001298 (450764); CNddU (452052); 2'-DEOXYURIDINE 3'-MONOPHOSPHATE (449392); [5-(2,4-dioxopyrimidin-1-yl)-3-hydroxy-4-methoxy-oxolan-2-yl]methoxyphosphonic acid (3908006); AIDS 160793 (500119); MOLI000591 (450214); NSC521272 (351357); AIDS060390 (469768); O2'-METHYLURIDINE 5'-MONOPHOSPHATE (5289071); 3'-Deoxyuridine-5'-triphosphate (149454); NSC258371 (318791); [5-(2,4-dioxopyrimidin-1-yl)-2-(hydroxymethyl)oxolan-3-yl]oxyphosphonic acid (3392033); AIDS 181187 (509512); 2'-Deoxyuridine 5'-monophosphate (446899); Novuridine (55262); UTP.γ.S (5311494); UDP.β.S (5310952); disodium 1-[3,4-dihydroxy-5-(phosphonatooxymethyl)oxolan-2-yl]pyrimidine-2,4-dione (3406450); VaraU (126501); Disodium UMP (18816); AIDS060438 (469809).

### HYDROCARBYL GROUP

The term "hydrocarbyl group" means a group that comprises at least carbon and hydrogen. Other atoms may be present such as N or O or S. The hydrocarbyl group may be linear or branched or cyclic. The hydrocarbyl group may be saturated or unsaturated. In some instances, two or more of the hydrocarbyl groups may be fused together - so as to form a single chain - that may be branched and/or saturated or unsaturated.

Representative hydrocarbyl groups may include: cycloimido groups, heterocycloimido groups, substituted amidino groups, heterocycloamidino groups, alkylcarbonylamino groups, alkyloxycarbonylamino groups, aminoalkyloxycarbonylamino groups, arylcarbonylamino groups, substituted aminocarbonyl groups, alkoxycarbonyl groups, heteroaryl groups, cycloimido groups, heterocycloimido groups, substituted amidino groups, heterocycloamidino groups, substituted alkylcarbonylamino groups, alkyloxycarbonylamino groups, aminoalkyloxycarbonylamino groups, arylcarbonylamino groups, substituted aminocarbonyl groups, substituted alkoxycarbonyl groups - each of which can be further substituted as will be apparent to those having skill in the organic and medicinal chemistry arts in conjunction with the disclosure herein.

As used herein, the hydrocarbyl groups - such as the heterocyclo groups - can be further substituted and may be attached at various positions as will be apparent to those having skill in the organic and medicinal chemistry arts in conjunction with the disclosure herein.

Preferably at least one of the hydrocarbyl gropus is a heterocyclo groups - can be further substituted and may be attached at various positions as will be apparent to those having skill in the organic and medicinal chemistry arts in conjunction with the disclosure herein.

One or more of the hydrocarbyl groups may be optionally substituted. "Optionally substituted" refers to the replacement of hydrogen with a monovalent or divalent radical. Suitable substitution groups include, for example, hydroxyl, nitro, amino, imino, cyano, halo, thio, thioamido, amidino, imidino, oxo, oxamidino, methoxamidino, imidino, guanidino, sulfonamido, carboxyl, formyl, loweralkyl, haloloweralkyl, loweralkoxy, haloloweralkoxy, loweralkoxyalkyl, alkylcarbonyl, arylcarbonyl, aralkylcarbonyl, heteroarylcarbonyl, heteroaralkylcarbonyl, alkylthio, aminoalkyl, cyanoalkyl, and the like.

The substitution group can itself be substituted. The group substituted onto the substitution group can be carboxyl, halo, nitro, amino, cyano, hydroxyl, loweralkyl, loweralkoxy, aminocarbonyl,-SR, thioamido,-SO3H,-SO2R or cycloalkyl, where R is typically hydrogen, hydroxyl or loweralkyl.

When the substituted substituent includes a straight chain group, the substitution can occur either within the chain (e. g., 2-hydroxypropyl, 2-aminobutyl, and the like) or at the chain terminus (e. g., 2-hydroxyethyl, 3-cyanopropyl, and the like). Substituted substitutents can be straight chain, branched or cyclic arrangements of covalently bonded carbon or heteroatoms.

One or more of the hydrocarbyl groups may be or may comprise an alkyl group - which may be saturated or unsaturated (e.g. an alkylene group) - and may be linear or branched. In some embodiments, preferably the alkyl group is a lower alkyl group.

The term "Lower alkyl" as used herein refers to branched or straight chain alkyl groups comprising one to ten carbon atoms that are unsubstituted or substituted, e. g., with one or more halogen, hydroxyl or other groups, including, e. g., methyl, ethyl, propyl, isopropyl, n-butyl, t-butyl, neopentyl, trifluoromethyl, pentafluoroethyl and the like.

One or more of the hydrocarbyl groups may be or may comprise an alkylenyl group. The term "alkylenyl" refers to a divalent straight chain or branched chain saturated aliphatic radical having from 1 to 20 carbon atoms. Typical alkylenyl groups employed in compounds are loweralkylenyl groups that have from 1 to about 6 carbon atoms in their backbone." Alkenyl" refers herein to straight chain, branched, or cyclic radicals having one or more double bonds and from 2 to 20 carbon atoms.

One or more of the hydrocarbyl groups may be or may comprise an alkynyl group. The term "alkynyl" refers herein to straight chain, branched, or cyclic radicals having one or more triple bonds and from 2 to 20 carbon atoms.

One or more of the hydrocarbyl groups may be or may comprise an alkoxy group - such as a lower alkoxy group. The term "lower alkoxy" as used herein refers to RO- wherein R is loweralkyl. Representative examples of lower alkoxy groups include methoxy, ethoxy, t-butoxy, trifluoromethoxy and the like.

One or more of the hydrocarbyl groups may be or may comprise a cycloalkyl group. The term "cycloalkyl" refers to a mono-or polycyclic, heterocyclic or carbocyclic alkyl substituent. Typical cycloalkyl substituents have from 3 to 8 backbone (i. e., ring) atoms in which each backbone atom is either carbon or a heteroatom. The term "heterocycloalkyl" refers herein to cycloalkyl substituents that have from 1 to 5, and more typically from 1 to 4 heteroatoms in the ring structure. Suitable heteroatoms employed in compounds are nitrogen, oxygen, and sulfur. Representative heterocycloalkyl moieties include, for example, morpholino, piperazinyl, piperadinyl and the like.

One or more of the hydrocarbyl groups may be or may comprise a carbocycloalkyl group. Carbocycloalkyl groups are cycloalkyl groups in which all ring atoms are carbon. When used in connection with cycloalkyl substituents, the term "polycyclic" refers herein to fused and non-fused alkyl cyclic structures.

One or more of the hydrocarbyl groups may comprise a halo group. The term "halo" refers herein to a halogen radical, such as fluorine, chlorine, bromine or iodine.

One or more of the hydrocarbyl groups may be or may comprise a haloalkyl group. The term "haloalkyl" refers to an alkyl radical substituted with one or more halogen atoms.

One or more of the hydrocarbyl groups may be or may comprise a haloloweralkyl group. The term "haloloweralkyl" refers to a loweralkyl radical substituted with one or more halogen atoms.

One or more of the hydrocarbyl groups may be or may comprise a haloalkoxy group. The term "haloalkoxy" refers to an alkoxy radical substituted with one or more halogen atoms.

One or more of the hydrocarbyl groups may be or may comprise a haloloweralkoxy group. The term "haloloweralkoxy" refers to a loweralkoxy radical substituted with one or more halogen atoms.

One or more of the hydrocarbyl groups may be or may comprise an aryl group. The term "aryl" refers to monocyclic and polycyclic aromatic groups having from 3 to 14 backbone carbon or hetero atoms, and includes both carbocyclic aryl groups and heterocyclic aryl groups.

One or more of the hydrocarbyl groups may be or may comprise a carbocyclic aryl group. Carbocyclic aryl groups are aryl groups in which all ring atoms in the aromatic ring are carbon.

One or more of the hydrocarbyl groups may be or may comprise an heteroaryl group. The term "heteroaryl" refers herein to aryl groups having from 1 to 4 heteroatoms as ring atoms in an aromatic ring with the remainder of the ring atoms being carbon atoms. When used in connection with aryl substituents, the term "polycyclic" refers herein to fused and non-fused cyclic structures in which at least one cyclic structure is aromatic, such as, for example, benzodioxozolo (which has a heterocyclic structure fused to a phenyl group, i. e., naphthyl, and the like.

Exemplary aryl moieties employed as substituents in the thymidine family members include phenyl, pyridyl, pyrimidinyl, thiazolyl, indolyl, imidazolyl, oxadiazolyl, tetrazolyl, pyrazinyl, triazolyl, thiophenyl, furanyl, quinolinyl, purinyl, naphthyl, benzothiazolyl, benzopyridyl, and benzimidazolyl, and the like.

One or more of the hydrocarbyl groups may be or may comprise an aralkyl group. The term "aralkyl" refers to an alkyl group substituted with an aryl group. Typically, aralkyl groups employed in thymidine family members have from 1 to 6 carbon atoms incorporated within the alkyl portion of the aralkyl group. Suitable aralkyl groups employed in thymidine family members include, for example, benzyl, picolyl, and the like.

One or more of the hydrocarbyl groups may comprise an amino group. The term "amino" refers herein to the group-NH2. The term "alkylamino" refers herein to the group-NRR'where R and R' are each independently selected from hydrogen or a lower alkyl. The term "arylamino" refers herein to the group-NRR'where R is aryl and R'is hydrogen, a lower alkyl, or an aryl. The term "aralkylamino" refers herein to the groupNRR'where R is a lower aralkyl and R'is hydrogen, a loweralkyl, an aryl, or a loweraralkyl.

One or more of the hydrocarbyl groups may be or may comprise an arylcycloalkylamino group. The term "arylcycloalkylamino" refers herein to the group, aryl-cycloalkyl-NH-, where cycloalkyl is a divalent cycloalkyl group. Typically, cycloalkyl has from 3 to 6 backbone atoms, of which, optionally 1 to about 4 are heteroatoms. The term "aminoalkyl" refers to an alkyl group that is terminally substituted with an amino group.

One or more of the hydrocarbyl groups may be or may comprise an alkoxyalkyl group. The term "alkoxyalkyl" refers to the group-alkl-O-alk2 where alkl is alkylenyl or alkenyl, and alk2 is alkyl or alkenyl.

One or more of the hydrocarbyl groups may be or may comprise an loweralkoxyalkyl group. The term "loweralkoxyalkyl" refers to an alkoxyalkyl where alkl is loweralkylenyl or loweralkenyl, and alk2 is loweralkyl or loweralkenyl.

One or more of the hydrocarbyl groups may be or may comprise an aryloxyalkyl group. The term "aryloxyalkyl" refers to the group-alkylenyl-O-aryl.

One or more of the hydrocarbyl groups may be or may comprise an aralkoxyalkyl group. The term "aralkoxyalkyl" refers to the group-alkylenyl-O-aralkyl, where aralkyl is a loweraralkyl.

One or more of the hydrocarbyl groups may be or may comprise an alkoxyalkylamino group. The term "alkoxyalkylamino" refers herein to the group-NR-(alkoxylalkyl), where R is typically hydrogen, loweraralkyl, or loweralkyl.

One or more of the hydrocarbyl groups may be or may comprise an aminoloweralkoxyalkyl group. The term "aminoloweralkoxyalkyl" refers herein to an aminoalkoxyalkyl in which the alkoxyalkyl is a loweralkoxyalkyl.

One or more of the hydrocarbyl groups may be or may comprise an aminocarbonyl group. The term "aminocarbonyl" refers herein to the group-C (O)-NH2.

One or more of the hydrocarbyl groups may be or may comprise a substituted aminocarbonyl group. The term "substituted aminocarbonyl" refers herein to the group-C (O)-NRR'where R is loweralkyl and R'is hydrogen or a loweralkyl.

One or more of the hydrocarbyl groups may be or may comprise an arylaminocarbonyl group. The term "arylaminocarbonyl" refers herein to the group-C (O)NRR'where R is an aryl and R'is hydrogen, loweralkyl or aryl.

One or more of the hydrocarbyl groups may be or may comprise an aralkylaminocarbonyl group. The term "aralkylaminocarbonyl" refers herein to the group-C (O)-NRR' where R is loweraralkyl and R'is hydrogen, loweralkyl, aryl, or loweraralkyl.

One or more of the hydrocarbyl groups may be or may comprise an aminosulfonyl group. The term "aminosulfonyl" refers herein to the group-S (0) 2-NH2."Substituted aminosulfonyl" refers herein to the group-S (O) 2-NRR' where R is loweralkyl and R'is hydrogen or a loweralkyl. The term "aralkylaminosulfonlyaryl" refers herein to the group - aryl-S (O) 2-NH-aralkyl, where the aralkyl is loweraralkyl.

One or more of the hydrocarbyl groups may comprise a carbonyl group. The term "carbonyl" refers to the divalent group-C (O)-.

One or more of the hydrocarbyl groups may comprise a carbonyloxy group. The term "carbonyloxy" refers generally to the group-C (O)-O-,. Such groups include esters, - C (O)-O-R, where R is loweralkyl, cycloalkyl, aryl, or loweraralkyl.

One or more of the hydrocarbyl groups may comprise a carbonyloxycycloalkyl group. The term "carbonyloxycycloalkyl" refers generally herein to both an"carbonyloxycarbocycloalkyl" and an"carbonyloxyheterocycloalkyl", i. e., where R is a carbocycloalkyl or heterocycloalkyl, respectively.

One or more of the hydrocarbyl groups may comprise an arylcarbonyloxy group. The term "arylcarbonyloxy" refers herein to the group C (O)-O-aryl, where aryl is a mono-or polycyclic, carbocycloaryl or heterocycloaryl.

One or more of the hydrocarbyl groups may comprise an aralkylcarbonyloxy group. The term "sulfonyl" refers herein to the group-S02-

The term "aralkylcarbonyloxy" refers herein to the group-C (O)-O-aralkyl, where the aralkyl is loweraralkyl.

One or more of the hydrocarbyl groups may comprise a sulfonyl group. The term "sulfonyl" refers herein to the group-S02-.

One or more of the hydrocarbyl groups may comprise an alkylsulfonyl group. The term "alkylsulfonyl" refers to a substituted sulfonyl of the structure-SO2R-in which R is alkyl. Alkylsulfonyl groups employed in thymidine family members are typically loweralkylsulfonyl groups having from 1 to 6 carbon atoms in its backbone structure. Thus, typical alkylsulfonyl groups employed in thymidine family members include, for example, methylsulfonyl (i. e., where R is methyl), ethylsulfonyl (i. e., where R is ethyl), propylsulfonyl (i. e., where R is propyl), and the like.

One or more of the hydrocarbyl groups may comprise an arylsulfonyl group. The term "arylsulfonyl" refers herein to the group-S02-aryl. The term "aralkylsulfonyl" refers herein to the group-S02-aralkyl, in which the aralkyl is loweraralkyl. The term "sulfonamido" refers herein to-SO2NH2.

One or more of the hydrocarbyl groups may comprise a carbonylamino group. The term "carbonylamino" refers to the divalent group-NH-C (O)in which the hydrogen atom of the amide nitrogen of the carbonylamino group can be replaced a loweralkyl, aryl, or loweraralkyl group. Such groups include moieties such as carbamate esters (-NH-C (O)-OR) and amides-NH-C (O)-O-R, where R is a straight or branched chain loweralkyl, cycloalkyl, or aryl or loweraralkyl.

One or more of the hydrocarbyl groups may comprise a loweralkylcarbonylamino group. The term "loweralkylcarbonylamino" refers to alkylcarbonylamino where R is a loweralkyl having from 1 to about 6 carbon atoms in its backbone structure.

One or more of the hydrocarbyl groups may comprise an arylcarbonylamino group. The term "arylcarbonylamino" refers to group-NH-C (O)-R where R is an aryl.

One or more of the hydrocarbyl groups may comprise an aralkylcarbonylamino group. The term "aralkylcarbonylamino" refers to carbonylamino where R is a lower aralkyl.

One or more of the hydrocarbyl groups may comprise a guanidino group or a guanidyl group. The terms "guanidino"or"guanidyl" refers to moieties derived from guanidine, H2N-C (=NH)-NH2. Such moieties include those bonded at the nitrogen atom carrying the formal double bond (the"2"-position of the guanidine, e. g., diaminomethyleneamino, (H2N) 2C=NH-) and those bonded at either of the nitrogen atoms carrying a formal single bond (the"1-"and/or"3"-positions of the guandine, e. g,

H2N-C (=NH)-NH-). The hydrogen atoms at any of the nitrogens can be replaced with a suitable substituent, such as loweralkyl, aryl, or loweraralkyl.

One or more of the hydrocarbyl groups may comprise an amidino group. The term "amidino" refers to the moieties R-C (=N)-NR'- (the radical being at the"N1"nitrogen) and R (NR') C=N- (the radical being at the"N2" nitrogen), where R and R'can be hydrogen, loweralkyl, aryl, or loweraralkyl.

Preferably, at least one of the hydrocarbyl groups comprises just C and H.

Preferably, the at least one of the hydrocarbyl groups which comprises just C and H is an alkyl group.

Preferably, the at least one of the hydrocarbyl groups which comprises just C and H is a C1-C6 alkyl group.

Preferably, the at least one of the hydrocarbyl groups which comprises just C and H is a linear alkyl group.

Preferably, the at least one of the hydrocarbyl groups which comprises just C and H is a linear C1-C6 alkyl group.

Preferably, the at least one of the hydrocarbyl groups which comprises just C and H is a saturated alkyl group.

Preferably, the at least one of the hydrocarbyl groups which comprises just C and H is a saturated C1-C6 alkyl group.

Preferably, the at least one of the hydrocarbyl groups which comprises just C and H is a linear and saturated alkyl group.

Preferably, the at least one of the hydrocarbyl groups which comprises just C and H is a linear and saturated C1-C6 alkyl group. An example of an unsaturated alkyl group is an alkylene group.

Preferably, the at least one of the hydrocarbyl groups which comprises just C and H is a C2 or C3 alkyl group.

Preferably, the at least one of the hydrocarbyl groups which comprises just C and H is a linear C2 or C3 alkyl group.

Preferably, the at least one of the hydrocarbyl groups which comprises just C and H is a saturated C2 or C3 alkyl group.

Preferably, the at least one of the hydrocarbyl groups which comprises just C and H is a linear and saturated C2 or C3 alkyl group.

Preferably, the at least one of the hydrocarbyl groups which comprises just C and H is a C3 alkyl group.

Preferably, the at least one of the hydrocarbyl groups which comprises just C and H is a linear C3 alkyl group.

Preferably, the at least one of the hydrocarbyl groups which comprises just C and H is a saturated C3 alkyl group.

Preferably, the at least one of the hydrocarbyl groups which comprises just C and H is a linear and saturated C3 alkyl group.

Preferably, the at least one of the hydrocarbyl groups which comprises just C and H is R2.

Preferably, at least one of the hydrocarbyl groups comprises at least C, H, N and O.

Preferably, at least two of the hydrocarbyl groups comprises at least C, H, N and O.

Preferably, the at least one of the hydrocarbyl groups which comprises at least C, H, N and O is a cyclic group.

Preferably, the at least one of the hydrocarbyl groups which comprises at least C, H, N and O is a substituted cyclic group.

Preferably, the at least one of the hydrocarbyl groups which comprises at least C, H, N and O is a substituted heterocyclic group.

### EXAMPLES

Where reference is made in the following Examples to "CloneXL Supplement", this should be read as reference to a composition containing uridine at 350.0 mg/L and thymidine at 12.0 mg/L.

### Example 1. Effect of Thymidine and Uridine on the Growth of Colonies of CHO-S Cells in Semisolid Culture

This experiment shows the effect of CloneXL Supplement containing thymidine and uridine on CHO-s cell growth and colony formation in semisolid media.

### CHO Cells

Suspension-adapted CHO cells used for this work are a clonal isolate, derived from Chinese Hamster Ovary (CHO K1) obtained from Invitrogen (Cat. No. 10743-029). Cells are routinely maintained in liquid culture on 75cm² tissue culture flasks (CellStar, Greiner Bio, Cat 659174) using a medium of the following composition (Formulation A, [mg/L]).

**Forumulation A**

| Component | Concentration (mg/l) |
|---|---|
| Calcium Chloride anhydrous | 200.00 |
| Potassium Chloride | 330.00 |
| Magnesium Sulphate anhydrous | 200.00 |
| Sodium Chloride | 45405.00 |
| Sodium Dihydrogen Phosphate · H₂O | 125.00 |
| Sodium Bicarbonate | 3024.00 |
| L-Arginine · HCl | 84.00 |
| L-Cysteine | 48.00 |
| Glycine | 30.00 |
| L-Histidine · HCl · H₂O | 42.00 |
| L-Isoleucine | 105.00 |
| L-Leucine | 105.00 |
| L-Lysine · HCl | 146.00 |
| L-Methionine | 30.00 |
| L-Phenylalanine | 66.00 |
| L-Serine | 42.00 |
| L-Threonine | 95.00 |
| L-Tryptophan | 16.00 |
| L-Tyrosine | 72.00 |
| L-Valine | 94.00 |
| D-Calcium-Pantothenic | 4.00 |
| Choline Chloride | 4.00 |
| Folic Acid | 4.00 |
| myo-Inositol | 7.20 |
| Nicotinamide | 4.00 |
| Pyridoxal - HCl | 4.00 |
| Riboflavin | 0.40 |
| Thiamine · HCl | 4.00 |
| D-Glucose anhydrous | 4500.0 |
| Sodium Pyruvate | 110.00 |
| HEPES | 5958.00 |
| L-Alanine | 25.00 |
| L-Asparagine H₂O | 28.40 |
| L-Aspartic Acid | 30.00 |
| L-Glutamic acid | 75.00 |
| L-Tyrosine disodium salt | 104.20 |
| Biotin | 0.013 |
| Vitamin B 12 | 0.013 |
| Sodium selenite | 0.017 |
| L praline | 50.00 |
| Ascorbic acid | 25.00 |
| Lipoic acid | 0.20 |
| Methyl linoleate | 0.088 |
| FeSO₄ | 1.00 |
| ZnSO₄ | 1.00 |
| CuSO₄ | 0.0025 |
| Ferric citrate | 2.00 |

Formulation A is supplemented with 8mM glutamine (GlutaMAX™-I, Cat. No. Invitrogen, 35050-038).

As an alternative, commercially available chemically-defined CHO media (e.g. GIBCO CD CHO medium, Invitrogen, Cat.No. 10743-011 or GIBCO CD CHO AGT granular medium, Invitrogen, Cat. No. 12490-017 or EX-CELL™ 302 Serum-Free Medium for CHO Cells, JRH 14324-500M or EX-CELL™ 302 Serum-Free Medium for CHO Cells, Dry Powder Medium (DPM), JRH, 24324-1L) may be used.

### CHO Cell Culture

CHO-S cultures are routinely passaged according to standard state of the art methods every 3 to 5 days, based on cell number and viability as determined by a hemocytometer count (Bright-Line™ Hemacytometer, Sigma, Z359629) and Trypan Blue vital stain (Trypan Blue solution 0.4%, Sigma-Aldrich, T8154).

For plating into semisolid media, cells are harvested on day 2 post-passaging.

5 mL of cells are passed through a sterile needle (BD Microlance, 304 432) and plastic syringe (BD 10mL syringe 3009112) to singularise the cells. Cells are counted as above to ascertain levels of viability are sufficiently high for plating (i.e. > 90% of cells viable).

### Semi-Solid Medium

The semisolid medium is prepared using CloneMatrix (Genetix, K8510) following the manufacturer's instructions. At room temperature, 0.5 volumes of 2x concentrated media Formulation A (see above), supplemented with 8mM glutamine (GlutaMAX™-I, Cat. No. Invitrogen, 35050-038) and 0.4 volumes of 2.5x concentrated CloneMatrix (see above) are mixed.

### Thymidine and Uridine Supplement

For test samples 0.02 volumes of a 50x concentrated CloneXL Supplement (containing uridine at 350.0 mg/L and thymidine at 12.0 mg/L) is added in addition to the other components to the media.

To obtain a seeding density of 1000 cells/ml a volume of singularised cells in liquid culture media as determined from the cell counts obtained (see above) is added to the two preparations of semisolid media (plus CloneXL Supplement and un-supplemented negative control).

Cells and semisolid media are mixed in a 50ml sterile tube (Fisher, Cat. No. 05-539-8) by gently inverting several times. Cells are plated by pouring approximately 2ml of media with cells into each well of a 6 well plate (Greiner, Suspension Culture Plate Cat.No. 657 185).

The semi solid cultures are incubated (Woolf Laboratories Galaxy F CO₂ Incubator) at 37 degrees centigrade and 5% CO₂.

### Results

Colony growth is monitored using a white light microscope and is documented in form of bitmap images.

Using these images the diameters of the obtained colonies are measured. Measurements are also taken for the diameters of ca. 10 single cells. The volume is calculated by using the formula v=(Π*d³)/6. The colony volume is here expressed as a multiple of a cell volume.

The results are shown in Figure 1 and Figure 2. The colony volumes are derived from diameters recorded on day 6 and day 10 post-plating and expressed as multiples of an average cell volume.

The presence of CloneXL containing uridine and thymidine in semi solid culture media enhances cell growth and colony formation of suspension-adapted CHO cells by an average factor of 3 by day 6.

By day 10 post-plating colonies grown in the presence of CloneXL Supplement containing uridine and thymidine are on average 44 times larger than colonies obtained in un-supplemented media.

This represents a time saving benefit in the process of clone selection, as colonies can be screened and picked at an earlier stage post-plating.

### Example 2. Effect of Thymidine and Uridine on the Growth of Colonies of CHO-s Cells in Semisolid Culture (Larger Scale)

A larger experiment is conducted in duplicate.

Cells are plated in four different preparations of semisolid media in both experiments conducted. These are; a) additive-free semisolid media, b) semisolid media with CloneXL Supplement, c) semisolid media spiked with 205 conditioned medium, d) semisolid media spiked with 20% conditioned medium and additional CloneXL Supplement. CloneXL Supplement contains uridine at 350.0 mg/L and thymidine at 12.0 mg/L.

Suspension-adapted CHO cells used for this work are a clonal isolate, derived from Chinese Hamster Ovary (CHO K1) obtained from Invitrogen (Cat. No. 10743-029). Cells are routinely maintained in liquid culture on 75cm² tissue culture flasks (CellStar, Greiner Bio, Cat 659174) using a media of the following composition (Formulation B, [mg/L]):

**Formulation B**

| Component | Concentration (mg/l) |
|---|---|
| Calcium Chloride anhydrous | 200.00 |
| Potassium Chloride | 330 |
| Magnesium Sulphate anhydrous | 200.00 |
| Sodium Chloride | 45405 |
| Sodium Dihydrogen Phosphate H2O | 125.00 |
| Sodium Bicarbonate | 3024.00 |
| L-Arginine HCl | 84.00 |
| L-Cysteine | 48.00 |
| Glycine | 30.00 |
| L-Histidine · HCl · H2O | 42.00 |
| L-Isoleucine | 105.00 |
| L-Leucine | 105.00 |
| L-Lysine · HCl | 146.00 |
| L-Methionine | 30.00 |
| L-Phenylalanine | 66.00 |
| L-Serine | 52.00 |
| L-Threonine | 95.00 |
| L-Tryptophan | 16.00 |
| L-Tyrosine | 72.00 |
| L-Valine | 94.00 |
| D-Calcium-Pantothenic | 4.00 |
| Choline Chloride | 4.00 |
| Folic Acid | 4.00 |
| myo-Inositol | 7.20 |
| Nicotinamide | 4.00 |
| Pyridoxal · HCl | 4.00 |
| Riboflavin | 0.40 |
| Thiamine · HCl | 4.00 |
| D-Glucose anhydrous | 4500.0 |
| Sodium Pyruvate | 110.00 |
| HEPES | 5958.00 |
| L-Alanine | 34.00 |
| L-Asparagine H₂O | 113.62 |
| L-Aspartic Acid | 43.00 |
| L-Glutamic acid | 150.00 |
| L-Tyrosine disodium salt | 104.20 |
| Biotin | 0.013 |
| Vitamin B 12 | 0.013 |
| Sodium selenite | 0.017 |
| L praline | 66.50 |
| Ascorbic acid | 25.00 |
| Lipoic acid | 0.20 |
| Methyl linoleate | 0.088 |
| FeSO₄ | 1.00 |
| ZnSO₄ | 1.00 |
| CuSO₄ | 0.0025 |
| Ferric citrate | 2.00 |
| Adenosine | 7.00 |
| Guanosine | 7.00 |
| Cytidine | 7.00 |

supplemented with 8mM glutamine (GlutaMAX™-I, Cat. No. Invitrogen, 35050-038).

CHO-S cultures are routinely passaged according to standard state of the art methods every 3 to 5 days, based on cell number and viability as determined by a hemocytometer count (Bright-Line^{™} Hemacytometer, Sigma, Z359629) and Trypan Blue vital stain (Trypan Blue solution 0.4%, Sigma-Aldrich, T8154)

For plating into semisolid media, cells are harvested on day 2 post-passaging.

5 mL of cells is passed through a sterile needle (BD Microlance, 304 432) and plastic syringe (BD 10mL syringe 3009112) to singularise the cells. Cells are counted as above to ascertain levels of viability are sufficiently high for plating (i.e. > 90% of cells viable).

The semisolid medium is prepared using CloneMatrix (Genetix, K8510) following the manufacturer's instructions. At room temperature, 0.5 volumes of 2x concentrated media Formulation B (see above), supplemented with 8mM glutamine (GlutaMAX™-I, Cat. No. Invitrogen, 35050-038) and 0.4 volumes of 2.5x concentrated CloneMatrix (see above) are mixed.

For test samples 0.02 volumes of a 50x concentrated CloneXL Supplement (containing uridine at 350.0 mg/L and thymidine 12.0 mg/L) is added in addition to the other components to the media.

As an additional condition, semisolid medium is also enriched by adding 0.2 volumes of a conditioned liquid medium.

Conditioned medium is obtained from the routinely maintained culture flask (see above) by sterile-filtering 20mL of liquid culture harvested on day 2 post-passaging through a 2um pore-sized syringe filter.

To obtain a seeding density of 1000 cells/ml a volume of singularised cells in liquid culture media as determined from the cell counts obtained (see above) is added to the two preparations of semisolid media (plus CloneXL Supplement containing uridine at 350.0 mg/L and thymidine at 12.0 mg/L and un-supplemented negative control).

Cells and semisolid media are mixed in a 50ml sterile tube (Fisher, Cat. No. 05-539-8) by gently inverting several times. Cells are plated by pouring approximately 2ml of media with cells into each well of a 6 well plate (Greiner, Suspension Culture Plate Cat.No. 657 185).

The semi solid cultures are incubated (Woolf Laboratories Galaxy F CO₂ Incubator) at 37 degrees centigrade and 5% CO₂.

### Results

Colony growth is monitored using a white light microscope and is documented in form of bitmap images.

Using these images the diameters of the obtained colonies are measured. Measurements are also taken for the diameters of ca. 10 single cells. The volume is calculated by using the formula v=(Π*d³)/6. The colony volume is here expressed as a multiple of a cell volume.

The results are shown in Figure 3. In both independent experiments, the addition of CloneXL containing thymidine and uridine to the conditioned as well as the unconditioned semi solid culture media had a marked effect on colony size by day 6 post-plating.

This is statistically significant in 3 out of four comparisons, despite the relatively small sample size.

This confirms that CloneXL containing thymidine and uridine has a time saving benefit in the process of clone selection.

### Example 3. Effect of Thymidine and Uridine on Growth of Suspension-Adapted CHO Cells Picked from Semisolid Media Into Small Scale Liquid Culture

This experiment was conducted to quantify the effect of CloneXL (containing uridine at 350.0 mg/L and thymidine at 12.0 mg/L) on CHO cell outgrowth after automated colony picking of the ClonePix FL.

Suspension-adapted CHO cells used for this work are a clonal isolate, derived from Chinese Hamster Ovary (CHO K1) obtained from Invitrogen (Cat. No. 10743-029). Cells are routinely maintained in liquid culture on 75cm² tissue culture flasks (CellStar, Greiner Bio, Cat 659174) using a media of the following composition (Formulation B, [mg/L]);

As an alternative, commercially available chemically-defined CHO media (e.g. GIBCO CD CHO medium, Invitrogen, Cat.No. 10743-011 or GIBCO CD CHO AGT granular medium, Invitrogen, Cat. No. 12490-017 or EX-CELL™ 302 Serum-Free Medium for CHO Cells, JRH 14324-500M or EX-CELL™ 302 Serum-Free Medium for CHO Cells, Dry Powder Medium (DPM), JRH, 24324-1L) may be used.

CHO-S cultures are routinely passaged according to standard state of the art methods every 3 to 5 days, based on cell number and viability as determined by a hemocytometer count (Bright-Line™ Hemacytometer, Sigma, Z359629) and Trypan Blue vital stain (Trypan Blue solution 0.4%, Sigma-Aldrich, T8154)

For plating into semisolid media, cells are harvested on day 2 post-passaging.

5 mL of cells is passed through a sterile needle (BD Microlance, 304 432) and plastic syringe (BD 10mL syringe 3009112) to singularise the cells. Cells are counted as above to ascertain levels of viability are sufficiently high for plating (i.e. > 90% of cells viable).

The semisolid medium is prepared using CloneMatrix (Genetix, K8510) following the manufacturer's instructions. At room temperature, 0.5 volumes of 2x concentrated media Formulation B (see above), supplemented with 8mM glutamine (GlutaMAX™-I, Cat. No. Invitrogen, 35050-038), 0.4 volumes of 2.5x concentrated CloneMatrix (see above), and 0.02 volumes of a 50x concentrated CloneXL Supplement (containing uridine at 350.0 mg/L and thymidine at 12.0 mg/L) are mixed.

To obtain a seeding density of 1000 cells/ml a volume of singularised cells in liquid culture media as determined from the cell counts obtained (see above) is added to the semisolid media.

Cells and semisolid media are mixed in a 50ml sterile tube (Fisher, Cat. No. 05-539-8) by gently inverting several times. Cells are plated by pouring approximately 2ml of media with cells into each well of a 6 well plate (Greiner, Suspension Culture Plate Cat.No. 657 185).

The semi solid cultures are incubated (Woolf Laboratories Galaxy F CO₂ Incubator) at 37 degrees centigrade and 5% CO₂.

Plates are imaged and colonies detected in the white light mode on the ClonePix FL system (Genetix) to identify colonies suitable for automated picking.

A total of 36 colonies are picked and transferred into wells of a 96 well destination plate containing media Formulation B (see above) supplemented with 8mM glutamine (GlutaMAX™-I, Cat. No. Invitrogen, 35050-038).

The liquid media in one half of the wells of this destination plate also contained the CloneXL Supplement containing thymidine and uridine added to a 1x final concentration.

After the automated picking and dispensing of CHO colonies into the destination plate, the picked cells are incubated as described above for a period of 8 days.

The destination plate is imaged in Fast Scan mode on the CloneSelect Imager (Genetix) immediately after picking and after 1, 2, 5, 6, 7, and 8 days of incubation.

The images of all wells obtained immediately after picking are screened to ascertain the presence of cells (i.e. successful automated picking and dispensing). Confluence readings for all wells are obtained and plotted in order of the level of confluence reached by day 8 post-incubation for all wells containing the CloneXL Supplement containing thymidine and uridine vs. cells grown in un-supplemented media.

### Results

In the presence of CloneXL Supplement containing thymidine and uridine, the recovery rate of CHO cells picked from semi solid media is boosted significantly.

78% of wells supplemented with CloneXL containing thymidine and uridine reached levels of confluence >10% by day 8 post-picking.

This is 2.6-fold higher than the outgrowth obtained in the absence of CloneXL Supplement containing thymidine and uridine, where 60% of wells did not display sufficient cell growth (i.e. confluence levels of at least 10%).

This is a crucial benefit of CloneXL containing thymidine and uridine in the process of clone selection and colony picking.

### Example 4. Effect of Thymidine and Uridine on the Plating Efficiency of Suspension-Adapted CHO Into Semisolid Media

The plating into semisolid media involves a fairly lengthy succession of various procedures all carried out at room temperature, which is stressful for the cells and results in varying degrees of loss of viability, otherwise expressed as plating efficiency. Suspension-adapted CHO cells display high sensitivity to all stresses posed during handling and require great care during all cell culture procedures.

Suspension-adapted CHO cells used for this work are a clonal isolate, derived from Chinese Hamster Ovary (CHO K1) obtained from Invitrogen (Cat. No. 10743-029). Cells are routinely maintained in liquid culture on 75cm² tissue culture flasks (CellStar, Greiner Bio, Cat 659174) using a media of the following composition (Formulation A, [mg/L]);

As an alternative, commercially available chemically-defined CHO media (e.g. GIBCO CD CHO medium, Invitrogen, Cat.No. 10743-011 or GIBCO CD CHO AGT granular medium, Invitrogen, Cat. No. 12490-017 or EX-CELL™ 302 Serum-Free Medium for CHO Cells, JRH 14324-500M or EX-CELL™ 302 Serum-Free Medium for CHO Cells, Dry Powder Medium (DPM), JRH, 24324-1L) may be used.

CHO-S cultures are routinely passaged according to standard state of the art methods every 3 to 5 days, based on cell number and viability as determined by a hemocytometer count (Bright-Line™ Hemacytometer, Sigma, Z359629) and Trypan Blue vital stain (Trypan Blue solution 0.4%, Sigma-Aldrich, T8154)

For plating into semisolid media, cells are harvested on day 2 post-passaging.

5 mL of cells is passed through a sterile needle (BD Microlance, 304 432) and plastic syringe (BD 10mL syringe 3009112) to singularise the cells. Cells are counted as above to ascertain levels of viability are sufficiently high for plating (i.e. > 90% of cells viable).

The semisolid medium is prepared using CloneMatrix (Genetix, K8510) following the manufacturer's instructions. At room temperature, 0.5 volumes of 2x concentrated media Formulation A (see above), supplemented with 8mM glutamine (GlutaMAX™-I, Cat. No. Invitrogen, 35050-038) and 0.4 volumes of 2.5x concentrated CloneMatrix (see above) are mixed.

For test samples 0.02 volumes of a 50x concentrated CloneXL Supplement (containing uridine at 350.0 mg/L and thymidine at 12.0 mg/L) is added in addition to the other components to the media.

To obtain a seeding density of 1000 cells/ml a volume of singularised cells in liquid culture media as determined from the cell counts obtained (see above) is added to the two preparations of semisolid media (plus CloneXL Supplement containing uridine at 350.0 mg/L and thymidine at 12.0 mg/L and un-supplemented negative control).

Cells and semisolid media are mixed in a 50ml sterile tube (Fisher, Cat. No. 05-539-8) by gently inverting several times. Cells are plated by pouring approximately 2ml of media with cells into each well of a 6 well plate (Greiner, Suspension Culture Plate Cat.No. 657 185). One whole dish of 6 wells is plated for each condition; un-supplemented semi solid media and media plus CloneXL supplement (containing uridine at 350.0 mg/L and thymidine at 12.0 mg/L).

The semi solid cultures are incubated (Woolf Laboratories Galaxy F CO₂ Incubator) at 37 degrees centigrade and 5% CO₂.

Plates are imaged in Fast Scan mode on the CloneSelect Imager (Genetix) on day 14 post-plating and colonies consisting of more than 16 cells are counted and ranked as follows;
- colonies resulting from 4 to 5 cell divisions, i.e. consisting of 16 to 32 cells are denoted as 'small',
- colonies resulting from 6 to 9 cell divisions, i.e. larger than 32, and smaller than 512 cells are denoted as 'average', and
- colonies resulting from more than 10 cell divisions, i.e. larger than 512 cells are denoted as 'large'.

### Results

In the six wells plated with cells in the absence of the CloneXL Supplement containing thymidine and uridine, a total of five 'small' colonies are detected. This equals a plating efficiency of 0.04%.

The sum of 'small', 'average', and 'large' colonies obtained in the six wells supplemented CloneXL Supplement containing thymidine and uridine is 64, which equals a ca. 26 times improved plating efficiency of 1.07%.

The survival of CHO-s cells and ability to form colonies is greatly improved in the presence of CloneXL Supplement containing thymidine and uridine.

At this delicate stage of the process of clone selection this represents a major benefit.

### Example 5. Effect of Thymidine and Uridine on Long-Term Cell Growth and Cell Viability

Two independent experiments are conducted to determine the long-term growth characteristics suspension-adapted CHO cells cultured in TC flasks in the presence and absence of the CloneXL Supplement containing thymidine and uridine.

Suspension-adapted CHO cells used for this work are a clonal isolate, derived from Chinese Hamster Ovary (CHO K1) obtained from Invitrogen (Cat. No. 10743-029). Cells are routinely maintained in liquid culture on 75cm² tissue culture flasks (CellStar, Greiner Bio, Cat 659174) using a media of the following composition (Formulation A, [mg/L]);

As an alternative, commercially available chemically-defined CHO media (e.g. GIBCO CD CHO medium, Invitrogen, Cat.No. 10743-011 or GIBCO CD CHO AGT granular medium, Invitrogen, Cat. No. 12490-017 or EX-CELL™ 302 Serum-Free Medium for CHO Cells, JRH 14324-500M or EX-CELL™ 302 Serum-Free Medium for CHO Cells, Dry Powder Medium (DPM), JRH, 24324-1L) may be used.

CHO-S cultures are routinely passaged according to standard state of the art methods every 3 to 5 days, based on cell number and viability as determined by a hemocytometer count (Bright-Line™ Hemacytometer, Sigma, Z359629) and Trypan Blue vital stain (Trypan Blue solution 0.4%, Sigma-Aldrich, T8154)

In two independent repeats of this experiment, cells are passaged as described above into 50 ml of media Formulation A (see above) supplemented with 8mM glutamine (GlutaMAX™-I, Cat. No. Invitrogen, 35050-038) in 75cm² tissue culture flasks (CellStar, Greiner Bio, Cat 659174) to a density of 1x10⁴ cells/ml. A second flask contained media supplemented with CloneXL (1x final concentration) and is inoculated as described above.

During two independent repeats of this experiment, flasks are incubated (Woolf Laboratories Galaxy F CO₂ Incubator) at 37 degrees centigrade and 5% CO₂.

Cell growth and viability are checked by hemocytometer cell counts and Trypan Blue vital stain (as above) on days 14 and 19 post-seeding, respectively.

### Results

In the flasks supplemented with CloneXL containing uridine and thymidine, increased final cell densities and improved viability are observed.

The percentage of viability is increased in the presence of CloneXL containing thymidine and uridine compared to the un-supplemented culture by 5.9 % (75.9% vs. 81.5% in first repeat) and 11.6% (25.0% vs. 36.6%), respectively.

This is clear evidence that cultures supplemented with CloneXL containing thymidine and uridine sustain increased levels of cell growth and viability over prolonged periods of cell culture.

This is potentially highly beneficial for large scale culture methods and for the production of cell products.

### Example 6. Effect of Thymidine and Uridine on Growth of Suspension-Adapted CHO Cells in Liquid Culture Using Different Commercial CHO Media

An experiment is conducted to quantify the effect of CloneXL on CHO cell growth in liquid culture.

Suspension-adapted CHO cells used for this work are a clonal isolate, derived from Chinese Hamster Ovary (CHO K1) obtained from Invitrogen (Cat. No. 10743-029). Cells are obtained a frozen stocks and seeded into and adapted to two commercially available chemically defined CHO media for at least 8 rounds of passaging.

These media are GIBCO CD CHO medium (Invitrogen, Cat.No. 10743-011) (referred to as Media A below) and EX-CELL™ 302 Serum-Free Medium for CHO Cells (JRH 14324-500M) (referred to as Media B below), both supplemented with 8mM glutamine (GlutaMAX™-I, Cat. No. Invitrogen, 35050-038).

Cells are maintained in these media in liquid culture in 75cm² tissue culture flasks (CellStar, Greiner Bio, Cat 659174).

CHO-S cultures are passaged according to standard state of the art methods every 3 to 5 days, based on cell number and viability as determined by a hemocytometer count (Bright-Line™ Hemacytometer, Sigma, Z359629) and Trypan Blue vital stain (Trypan Blue solution 0.4%, Sigma-Aldrich, T8154)

Cells are seeded at a density of 1x10³ cells/ml into one flat-bottomed 96 well plate (Greiner Cellstar, Cat. No. 655 180) for each of the two media.

The media in one half of the wells also contained the CloneXL containing added to a 1x final concentration.

The culture plates are incubated (Woolf Laboratories Galaxy F CO₂ Incubator) at 37 degrees centigrade and 5% CO₂.

Cell growth is monitored by scanning in Fast Scan mode on the CloneSelect Imager (Genetix) immediately after plating and once daily thereafter.

Confluence readings for 48 wells per condition are obtained normalised to the level of confluence upon seeding and their mean and standard deviation calculated.

The results are shown in Figure 6.

Normalised confluence readings (Y-axis) obtained on day 6 are 2.9-fold increased due to the addition of CloneXL containing thymidine and uridine in Media A (GIBCO CD CHO medium, Invitrogen, Cat.No. 10743-011) and 3.7-fold increased due to the addition of CloneXL containing thymidine and uridine in Media B (EX-CELL™ 302 Serum-Free Medium for CHO Cells, JRH 14324-500M).

### Results

The 2.9-fold and 3.7-fold increased confluence due to the addition of CloneXL containing thymidine and uridine in two different commercial CHO media shows that the benefit of supplementation with CloneXL containing thymidine and uridine is independent and in addition to the cell culture media used.

Each of the applications and patents mentioned in this document, and each document cited or referenced in each of the above applications and patents, including during the prosecution of each of the applications and patents ("application cited documents") and any manufacturer's instructions or catalogues for any products cited or mentioned in each of the applications and patents and in any of the application cited documents, are hereby incorporated herein by reference. Furthermore, all documents cited in this text, and all documents cited or referenced in documents cited in this text, and any manufacturer's instructions or catalogues for any products cited or mentioned in this text, are hereby incorporated herein by reference.

Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments and that many modifications and additions thereto may be made. Furthermore, various combinations of the features of the following dependent claims can be made with the features of the independent claims.

## Claims

1. A method of growing an animal cell in a culture medium, in which the culture medium comprises thymidine at 0.2 to 7 mg/l and uridine at 5 to 10 mg/l, in which the growth or viability of the animal cell is increased as a result of the thymidine and uridine in the cell culture medium, wherein the cell culture medium is substantially free of serum, and wherein the animal cell is a Chinese Hamster Ovary (CHO) cell.

2. The method according to Claim 1, in which the method comprises supplementing a basal medium with a supplement comprising thymidine and uridine to produce the cell culture medium.

3. The method according to Claim 2, in which the basal medium is a minimal medium.

4. The method according to Claim 1 or 3, in which the basal medium substantially lacks thymidine.

5. The method according to any preceding claim, in which the cell culture medium is a chemically defined medium.

6. The method according to any preceding claim, in which the cell culture medium comprises thymidine at 0.24 mg/l.

7. The method according to any preceding claim, in which the cell culture medium comprises uridine at 7 mg/ll.

8. The method according to any preceding claim, in which the cell culture medium further comprises conditioned medium.

9. The method according to any preceding claim, in which the cell culture medium does not substantially comprise adenosine.

10. The method according to any preceding claim, in which the cell culture medium is a semi-solid medium.

11. The method according to any preceding claim, in which the semi-solid medium comprises methylcellulose.

12. The method according to any preceding claim, in which the cell culture medium is a liquid medium.

13. The method according to any preceding claim, in which cell growth or cell viability is enhanced by at least 50% compared to growth in a cell culture medium without an elevated concentration of thymidine and uridine.

14. The method according to any preceding claim, in which apoptosis of the animal cell in the cell culture medium is reduced to enhance cell viability.

15. The method according to any preceding claim, in which the animal cell is transformed with an expression vector, the expression vector being a member of a nucleic acid library.

16. The method according to any preceding claim, in which the animal cell expresses a heterologous or recombinant protein.

17. The method according to Claim 16, in which the heterologous or recombinant protein is a therapeutic protein or a therapeutic antibody.

18. Use of thymidine and uridine to enhance the growth or viability of a Chinese Hamster Ovary (CHO) cell in a semi-solid serum-free culture medium, wherein the thymidine is present at 0.2 to 7 mg/l and uridine is present at 5 to 10 mg/l.

19. A method of producing a recombinant protein, the method comprising culturing an animal cell which expresses the recombinant protein in a method according to any of Claims 1 to 17, wherein the animal cell is a Chinese Hamster Ovary (CHO) cell.

20. A method of increasing the yield of a recombinant protein from an animal cell, the method comprising enhancing the growth or viability of the animal cell by a method according to any of Claims 1 to 17, wherein the animal cell is a Chinese Hamster Ovary (CHO) cell.

21. The method according to Claim 20, in which the yield is increased by at least 10% compared to the yield from a culture medium without an elevated concentration of thymidine.

22. A serum-free cell culture medium comprising a growth enhancing concentration of thymidine and uridine, wherein thymidine is present at 0.2 to 7 mg/l and uridine is present at 5 to 10 mg/l.

23. A serum-free supplement or concentrate comprising thymidine and uridine, which when diluted, provides a cell culture medium according to Claim 22, or which is suitable for use in a method according to any of Claims 1 to 17.

24. A serum-free composition comprising 350 mg/l of uridine and 12 mg/l of thymidine.

25. A method of producing a recombinant protein, the method comprising culturing an animal cell which expresses the recombinant protein in a serum-free liquid medium comprising thymidine at 0.2 to 7 mg/l and uridine at 5 to 10 mg/l, wherein the animal cell is a Chinese Hamster Ovary (CHO) cell.

## Patentansprüche

1. Verfahren zum Züchten einer Tierzelle in einem Kulturmedium, bei welchem das Kulturmedium Thymidin zu 0,2 bis 7 mg/l und Uridin zu 5 bis 10 mg/l umfasst, bei welchem das Wachstum und die Lebensfähigkeit der Tierzelle als Ergebnis des Thymidins und Uridins in dem Zellkulturmedium gesteigert ist, wobei das Zellkulturmedium im Wesentlichen frei von Serum ist und wobei die Tierzelle eine Ovarzelle des chinesischen Hamsters (CHO-Zelle) ist.

2. Verfahren gemäß Anspruch 1, bei welchem das Verfahren das Ergänzen eines Basismediums mit einem Thymidin und Uridin enthaltenden Zusatz unter Erhalt des Zellkulturmediums umfasst.

3. Verfahren gemäß Anspruch 2, bei welchem das Basismedium ein Minimalmedium ist.

4. Verfahren gemäß Anspruch 1 oder 3, bei welchem es dem Basismedium im Wesentlichen an Thymidin fehlt.

5. Verfahren gemäß einem der vorangehenden Ansprüche, bei welchem das Zellkulturmedium ein chemisch definiertes Medium ist.

6. Verfahren gemäß einem der vorangehenden Ansprüche, bei welchem das Zellkulturmedium Thymidin zu 0,24 mg/l umfasst.

7. Verfahren gemäß einem der vorangehenden Ansprüche, bei welchem das Zellkulturmedium Uridin zu 7 mg/l umfasst.

8. Verfahren gemäß einem der vorangehenden Ansprüche, bei welchem das Zellkulturmedium weiter ein konditioniertes Medium umfasst.

9. Verfahren gemäß einem der vorangehenden Ansprüche, bei welchem das Zellkulturmedium im Wesentlichen kein Adenosin umfasst.

10. Verfahren gemäß einem der vorangehenden Ansprüche, bei welchem das Zellkulturmedium ein halbfestes Medium ist.

11. Verfahren gemäß einem der vorangehenden Ansprüche, bei welchem das halbfeste Medium Methylzellulose umfasst.

12. Verfahren gemäß einem der vorangehenden Ansprüche, bei welchem das Zellkulturmedium ein Flüssigmedium ist.

13. Verfahren gemäß einem der vorangehenden Ansprüche, bei welchem das Zellwachstum oder die Zelllebensfähigkeit um wenigstens 50% gesteigert ist im Vergleich zum Wachstum in einem Zellkulturmedium ohne erhöhte Konzentration von Thymidin und Uridin.

14. Verfahren gemäß einem der vorangehenden Ansprüche, bei welchem Apoptose der Tierzelle in dem Zellkulturmedium zur Verbesserung der Zelllebensfähigkeit reduziert ist.

15. Verfahren gemäß einem der vorangehenden Ansprüche, bei welchem die Tierzelle mit einem Expressionsvektor transformiert ist, wobei der Expressionsvektor ein Teil einer Nukleinsäurebibliothek ist.

16. Verfahren gemäß einem der vorangehenden Ansprüche, bei welchem die Tierzelle ein heterologes oder rekombinantes Protein exprimiert.

17. Verfahren gemäß Anspruch 16, wobei das heterologe oder rekombinante Protein ein therapeutisches Protein oder ein therapeutischer Antikörper ist.

18. Verwendung von Thymidin und Uridin zur Steigerung des Wachstums oder der Lebensfähigkeit von Ovarzellen des chinesischen Hamsters (CHO-Zellen) in einem halbfesten serumfreien Kulturmedium, wobei das Thymidin zu 0,2 bis 7 mg/l vorhanden ist und das Uridin zu 5 bis 10 mg/l vorhanden ist.

19. Verfahren zur Herstellung eines rekombinanten Proteins, wobei das Verfahren das Kultivieren einer Tierzelle, die das rekombinante Protein umfasst, in einem Verfahren gemäß einem der Ansprüche 1 bis 17 umfasst, wobei die Tierzelle eine Ovarzelle des chinesischen Hamsters (CHO-Zelle) ist.

20. Verfahren zur Steigerung der Ausbeute eines rekombinanten Proteins aus einer Tierzelle, wobei das Verfahren die Steigerung des Wachstums oder der Lebensfähigkeit der Tierzelle durch ein Verfahren gemäß einem der Ansprüche 1 bis 17 umfasst, wobei die Tierzelle eine Ovarzelle des chinesischen Hamsters (CHO-Zelle) ist.

21. Verfahren gemäß Anspruch 20, bei welchem die Ausbeute um wenigstens 10% gesteigert ist im Vergleich zur Ausbeute aus einem Kulturmedium ohne erhöhte Konzentration an Thymidin.

22. Serumfreies Zellkulturmedium, welches eine wachstumssteigernde Konzentration von Thymidin und Uridin umfasst, wobei Thymidin zu 0,2 bis 7 mg/l vorhanden ist und Uridin zu 5-10 mg/l vorhanden ist.

23. Serumfreier Zusatz oder Konzentrat, der/das Thymidin und Uridin umfasst, der/das beim Verdünnen ein Zellkulturmedium gemäß Anspruch 22 bereitstellt oder der/das zur Verwendung in einem Verfahren gemäß einem der Ansprüche 1 bis 17 geeignet ist.

24. Serumfreie Zusammensetzung, die 350 mg/l Uridin und 12 mg/l Thymidin umfasst.

25. Verfahren zur Herstellung eines rekombinanten Proteins, wobei das Verfahren das Kultivieren einer Tierzelle umfasst, die das rekombinante Protein in einem serumfreien Flüssigmedium exprimiert, das Thymidin zu 0,2 bis 7 mg/l und Uridin zu 5 bis 10 mg/l umfasst, wobei die Tierzelle eine Ovarzelle des chinesischen Hamsters (CHO-Zelle) ist.

## Revendications

1. Procédé pour cultiver une cellule animale dans un milieu de culture, où le milieu de culture comprend de la thymidine entre 0,2 et 7 mg/l et de l'uridine entre 5 et 10 mg/l, dans lequel la croissance ou la viabilité de la cellule animale est augmentée en conséquence de la thymidine et de l'uridine dans le milieu de culture cellulaire, où le milieu de culture cellulaire est essentiellement exempt de sérum, et où la cellule animale est une cellule d'ovaire de hamster chinois (CHO).

2. Procédé selon la revendication 1, où le procédé consiste à supplémenter un milieu basal avec un supplément comprenant de la thymidine et de l'uridine afin de produire le milieu de culture cellulaire.

3. Procédé selon la revendication 2, dans lequel le milieu basal est un milieu minimal.

4. Procédé selon la revendication 1 ou 3, dans lequel le milieu basal est essentiellement dépourvu de thymidine.

5. Procédé selon l'une quelconque des revendications précédentes, dans lequel le milieu de culture cellulaire est un milieu chimiquement défini.

6. Procédé selon l'une quelconque des revendications précédentes, dans lequel le milieu de culture cellulaire comprend de la thymidine à 0,24 mg/l.

7. Procédé selon l'une quelconque des revendications précédentes, dans lequel le milieu de culture cellulaire comprend de l'uridine à 7 mg/l.

8. Procédé selon l'une quelconque des revendications précédentes, dans lequel le milieu de culture cellulaire comprend en outre un milieu conditionné.

9. Procédé selon l'une quelconque des revendications précédentes, dans lequel le milieu de culture cellulaire ne comprend essentiellement pas d'adénosine.

10. Procédé selon l'une quelconque des revendications précédentes, dans lequel le milieu de culture cellulaire est un milieu semi-solide.

11. Procédé selon l'une quelconque des revendications précédentes, dans lequel le milieu semi-solide comprend de la méthylcellulose.

12. Procédé selon l'une quelconque des revendications précédentes, dans lequel le milieu de culture cellulaire est un milieu liquide.

13. Procédé selon l'une quelconque des revendications précédentes, dans lequel la croissance cellulaire ou la viabilité cellulaire est améliorée d'au moins 50 % par comparaison à une croissance dans un milieu de culture cellulaire ne contenant pas de concentration élevée de thymidine et d'uridine.

14. Procédé selon l'une quelconque des revendications précédentes, dans lequel une apoptose de la cellule animale dans le milieu de culture cellulaire est réduite afin d'améliorer la viabilité cellulaire.

15. Procédé selon l'une quelconque des revendications précédentes, dans lequel la cellule animale est transformée avec un vecteur d'expression, le vecteur d'expression étant un membre d'une banque d'acides nucléiques.

16. Procédé selon l'une quelconque des revendications précédentes, dans lequel la cellule animale exprime une protéine hétérologue ou recombinante.

17. Procédé selon la revendication 16, dans lequel la protéine hétérologue ou recombinante est une protéine thérapeutique ou un anticorps thérapeutique.

18. Utilisation de thymidine et d'uridine pour améliorer la croissance ou la viabilité d'une cellule d'ovaire de hamster chinois (CHO) dans un milieu de culture semi-solide exempt de sérum, où la thymidine est présente entre 0,2 et 7 mg/l et l'uridine est présente entre 5 et 10 mg/l.

19. Procédé de production d'une protéine recombinante, le procédé comprenant la culture d'une cellule animale qui exprime la protéine recombinante dans un procédé selon l'une quelconque des revendications 1 à 17, où la cellule animale est une cellule d'ovaire de hamster chinois (CHO).

20. Procédé pour augmenter le rendement d'une protéine recombinante à partir d'une cellule animale, le procédé comprenant l'amélioration de la croissance ou de la viabilité de la cellule animale par un procédé selon l'une quelconque des revendications 1 à 17, où la cellule animale est une cellule d'ovaire de hamster chinois (CHO).

21. Procédé selon la revendication 20, dans lequel le rendement est augmenté d'au moins 10 % par comparaison au rendement obtenu à partir d'un milieu de culture ne contenant pas de concentration élevée de thymidine.

22. Milieu de culture cellulaire exempt de sérum, comprenant une concentration de thymidine et d'uridine améliorant la croissance, où thymidine est présente entre 0,2 et 7 mg/l et uridine est présente entre 5 et 10 mg/l.

23. Supplément ou concentré exempt de sérum comprenant de la thymidine et de l'uridine qui, lorsqu'il est dilué, offre un milieu de culture cellulaire selon la revendication 22, ou qui est approprié pour être utilisé dans un procédé selon l'une quelconque des revendications 1 à 17.

24. Composition exempte de sérum comprenant 350 mg/l d'uridine et 12 mg/l de thymidine.

25. Procédé de production d'une protéine recombinante, le procédé comprenant la culture d'une cellule animale qui exprime la protéine recombinante dans un milieu liquide exempt de sérum comprenant de la thymidine entre 0,2 et 7 mg/l et de l'uridine entre 5 et 10 mg/l, où la cellule animale est une cellule d'ovaire de hamster chinois (CHO).
